# EUROPEAN PATENT APPLICATION

(11) **EP 4 650 607 A1**
(43) Date of publication of application: **19.11.2025**
(21) Application number: 23915828.0
(22) Date of filing: 28.12.2023
(51) Int. Cl.: F15B 11/08, C12Q 1/6869

(54) **FLUID PATH SYSTEM AND SEQUENCING SYSTEM**

(30) Priority: 13.01.2023 CN 202310078181
(71) Applicant: GeneMind Biosciences Company Limited, Shenzhen, Guangdong 518000 (CN)
(72) Inventor: HAN, Weichun, Shenzhen, Guangdong 518000 (CN); LAI, Lin, Shenzhen, Guangdong 518000 (CN); SU, Hangpeng, Shenzhen, Guangdong 518000 (CN); WU, Ping, Shenzhen, Guangdong 518000 (CN); WANG, Guangming, Shenzhen, Guangdong 518000 (CN)
(74) Representative: Schiweck Weinzierl Koch Patentanwälte Partnerschaft mbB
(86) International application number: PCT/CN2023/142602
(87) International publication number: WO 2024/149075

(57) **Abstract**

A fluid path system (1000) and a sequencing system. The fluid path system comprises a first storage device (100), a second storage (200), a rotary valve (300), a pump assembly (400) and a reaction apparatus (500), which are independent; one of the storage devices can be in communication with the rotary valve by means of a first flow path (10), the other storage device can be in communication with the rotary valve by means of a second flow path (11), and the rotary valve is connected to the reaction device by means of a third flow path (13); the pump assembly is connected to the rotary valve by means of a fourth flow path (12). The fluid path system is selectively in a first state or a second state; when the fluid path system is in the first state, the pump assembly drives fluid in one of the storage devices to enter the fourth flow path by means of the rotary valve; and when the fluid path system is in the second state, the pump assembly drives fluid in at least one other storage device to enter the reaction apparatus by means of the rotary valve. The fluid path system and the sequencing system comprising the fluid path system have both sequencing and cleaning functions.

## Description

The present application claims priority to Chinese Patent Application No. 202310078181.9 filed to China National Intellectual Property Administration on January. 13, 2023, and entitled "FLUIDIC SYSTEM AND SEQUENCING SYSTEM", which is incorporated herein by reference in its entirety.

### TECHNICAL FIELD

Embodiments of the present application relate to the technical field of gene sequencing, and in particular, to a fluidic system and a sequencing system.

### BACKGROUND

A fluidic system of a biochemical substance analysis instrument (such as a gene sequencer) is used for sequencing different reagents. After the completion of sequencing, in the fluidic system, reagents usually remain in a reagent needle for aspirating reagents or a tube through which reagents flow. If this part of the reagents is not cleaned in time and the instrument is not used for a period of time, the reagents remaining in the fluidic system may affect the performance of the instrument.

### SUMMARY

The present application provides a fluidic system and a sequencing system.

The fluidic system according to the embodiments of the present application includes:
a plurality of reservoirs;
a rotary valve, where one of the reservoirs is capable of being in communication with the rotary valve through a first flow path, the remaining reservoirs are capable of being in communication with the rotary valve through a second flow path, and the rotary valve is connected to a reaction device through a third flow path; and
a pump assembly, connected to the rotary valve through a fourth flow path.

The fluidic system is selectively in a first state or a second state. When the fluidic system is in the first state, the pump assembly drives liquid in one of the reservoirs to enter the fourth flow path through the rotary valve;

When the fluidic system is in the second state, the pump assembly drives liquid in at least one of the remaining reservoirs to enter the reaction device through the rotary valve.

In the fluidic system according to the embodiments of the present application, the fluidic system is configured to be able to switch between the first state and the second state, with the liquid in the fluidic system entering the fourth flow path or the reaction device in different states. When different liquids are stored in the reservoirs, the fluidic system can exhibit different functions. Further, when the reservoirs store a sequencing reagent and a cleaning solution, respectively, the fluidic system can exhibit both the sequencing and cleaning function, such that the fluidic system can be cleaned in time after the completion of sequencing, and there is no sequencing reagent remaining in the fluidic system, thereby ensuring the use performance of the instrument.

The sequencing system according to the embodiments of the present application includes the fluidic system described in the above embodiments.

The additional aspects and advantages of the present application will be partially set forth in the following description, and will partially become apparent from the following description or be appreciated by practice of the present application.

### BRIEF DESCRIPTION OF THE DRAWINGS

The aforementioned and/or additional aspects and advantages of the present application will become apparent and easily understood from the description of the embodiments with reference to the following drawings, in which:
FIG. 1 is a structural schematic view of a fluidic system according to the embodiments of the present application;
FIG. 2 is a schematic view of a partial structure of a rotary valve according to the embodiments of the present application;
FIG. 3 is a structural schematic view of a rotor according to the embodiments of the present application;
FIG. 4 is a structural schematic view of a stator according to the embodiments of the present application;
FIG. 5 is a structural schematic view of a stator according to the embodiments of the present application;
FIG. 6 is a structural schematic view of a rotor according to the embodiments of the present application;
FIG. 7 is a structural schematic view of a fluidic system according to the embodiments of the present application;
FIG. 8 is a schematic view of an assembly structure of a fixed frame, a reagent kit, and a cooling device according to the embodiments of the present application;
FIG. 9 is an exploded schematic view of the assembly structure in FIG. 8;
FIG. 10 is a schematic view of an assembly structure of a fixed frame and reagent needles according to the embodiments of the present application;
FIG. 11 is a schematic view of an assembly structure of a second reagent needle and a mounting base according to the embodiments of the present application;
FIG. 12 is a cross-sectional view of the assembly structure in FIG. 11 in a direction of a;
FIG. 13 is a schematic view of a partial structure of a fluidic system according to the embodiments of the present application;
FIG. 14 is a schematic view of a partial structure of a fluidic system according to the embodiments of the present application;
FIG. 15 is a structural schematic view of a cover assembly according to the embodiments of the present application;
FIG. 16 is a schematic view of a partial structure of a fluidic system according to the embodiments of the present application;
FIG. 17 is a schematic view of a partial structure of the assembly structure in FIG. 8;
FIG. 18 is a structural schematic view of a second detection assembly according to the embodiments of the present application;
FIG. 19 is a schematic view of a partial structure of the assembly structure in FIG. 8;
FIG. 20 is a structural schematic view of a bracket according to the embodiments of the present application; and
FIG. 21 is a structural schematic view of a lower plate according to the embodiments of the present application.

Description of reference numerals for main elements: fluidic system 1000; first reservoir 100; second reservoir 200; rotary valve 300; pump assembly 400; reaction device 500; first unit 501; first unit 502; first flow path 10; second flow path 11; fourth flow path 12; third flow path 13; first fluidic unit 600; second fluidic unit 700; first manifold block 800; first liquid inlet 810; second liquid inlet 820; first liquid outlet 830; second liquid outlet 840; third liquid outlet 850; fourth liquid outlet 860; pressure sensor 900; second manifold block 14; first common port 310; second common port 320; connection port 330; common port 301; first communication groove 340; second communication groove 350; stator 360; rotor 370; first groove 341; second groove 342; first end of the first groove 3410; second end of the first groove 3411; first end of the second groove 3420; second end of the second groove 3421; first end surface 361; second end surface 362; third end surface 371; fourth end surface 372; first end of the second communication groove 351; second end of the second communication groove 352; multi-way valve 15; first through port 150; second through port 151; third through port 152; first reagent needle 16; first driving mechanism 17; first type of reagent needle 160; second type of reagent needle 161; third type of reagent needle 162; third manifold block 18; liquid access port 19; fixed frame 170; movable member 171; first driving assembly 172; first plate 173; second plate 174; connecting post 175; first motor 1720; first lead screw 1721; second reagent needle 20; mounting base 21; guiding member 210; sliding member 211; first elastic member 22; pressing piece 1710; second elastic member 23; mounting hole 2110; needle body 201; flange 202; first detection assembly 24; first photoelectric switch 240; second photoelectric switch 241; support plate 176; light shielding member 1711; liquid collector 25; liquid inlet 250; cover assembly 26; guide rail 260; connecting frame 261; gland 262; first limiting position 2610; second limiting position 2611; groove 2613; elastic member 27; pulling device 28; drawer box 280; sliding rail 281; partition plate 2801; reagent kit poka-yoke block 2802; liquid collector poka-yoke block 2803; bracket 29; second driving mechanism 30; second motor 31; second lead screw 32; second detection assembly 40; third photoelectric switch 41; fourth photoelectric switch 42; first light blocking member 43; second light blocking member 44; first limiting block 290; second limiting block 291; cooling device 50; box body 51; chamber 510; through hole 292; lower plate 52; accommodating groove 520; water discharge hole 521; strip-shaped groove 523; reagent kit 60; liquid manifold block 70; liquid discharge tube 71; fifth photoelectric switch 45; third light blocking member 46.

### DETAILED DESCRIPTION

Embodiments of the present application are described in detail below, and the examples of the embodiments are shown in the drawings, throughout which identical or similar reference numerals represent identical or similar elements or elements having identical or similar functions. The embodiments described below with reference to the drawings are exemplary and are merely intended to explain the present application, and should not be construed as limiting the present application.

In the description of the present application, it should be understood that orientational or positional relationships indicated by terms such as "central", "longitudinal", "transverse", "length", "width", "thickness", "upper", "lower", "front", "rear", "left", "right", "vertical", "horizontal", "top", "bottom", "inner", "outer", "clockwise", or "counterclockwise", are those shown on the basis of the drawings, and are merely intended to facilitate and simplify the description rather than indicate or imply that the indicated device or element must have a specific orientation and be configured and operated according to the specific orientation. Such relationships should not be construed as limiting the present application. In addition, the terms "first" and "second" are used herein for descriptive purposes only and should not be construed as indicating or implying relative importance or implicitly indicating the number of technical features described. Therefore, features defined with "first" and "second" may explicitly or implicitly include one or more of the features. In the description of the present application, unless otherwise specifically defined, "a plurality of" means two or more than two.

In the description of the present application, it should be noted that unless otherwise clearly specified and defined, the terms "mount", "link", and "connect" should be interpreted in their broad sense. For example, the connection may be a fixed connection, detachable connection, or integral connection; a mechanical connection, electric connection, or communicative connection; or a direct connection, indirect connection through an intermediate, internal communication of two elements, or interaction between two elements. For those of ordinary skill in the art, the specific meanings of the aforementioned terms in the present application can be understood according to specific conditions.

In the present application, unless otherwise clearly specified and defined, a first feature being "above" or "below" a second feature may include that the first and second features are in direct contact and that the first and second features are not in direct contact but are in contact through an additional feature therebetween. Moreover, a first feature being "on", "over", and "above" a second feature includes that the first feature is right above or obliquely above the second feature, or simply means that the first feature is at a vertically higher position than the second feature. A first feature being "under", "beneath", and "below" a second feature includes that the first feature is right below or obliquely below the second feature, or simply means that the first feature is at a vertically lower position than the second feature.

The following disclosure provides many different embodiments or examples for implementing different structures of the present application. To simplify the disclosure of the present application, the components and arrangements of specific examples are described below. Certainly, the examples are merely exemplary and are not intended to limit the present application. In addition, the present application may repeat reference numbers and/or reference letters in different examples. Such repetition is intended for simplicity and clarity rather than for indicating the relationship between various embodiments and/or arrangements discussed. In addition, the present application provides examples of various specific processes and materials, but those of ordinary skill in the art will recognize the application of other processes and/or the use of other materials.

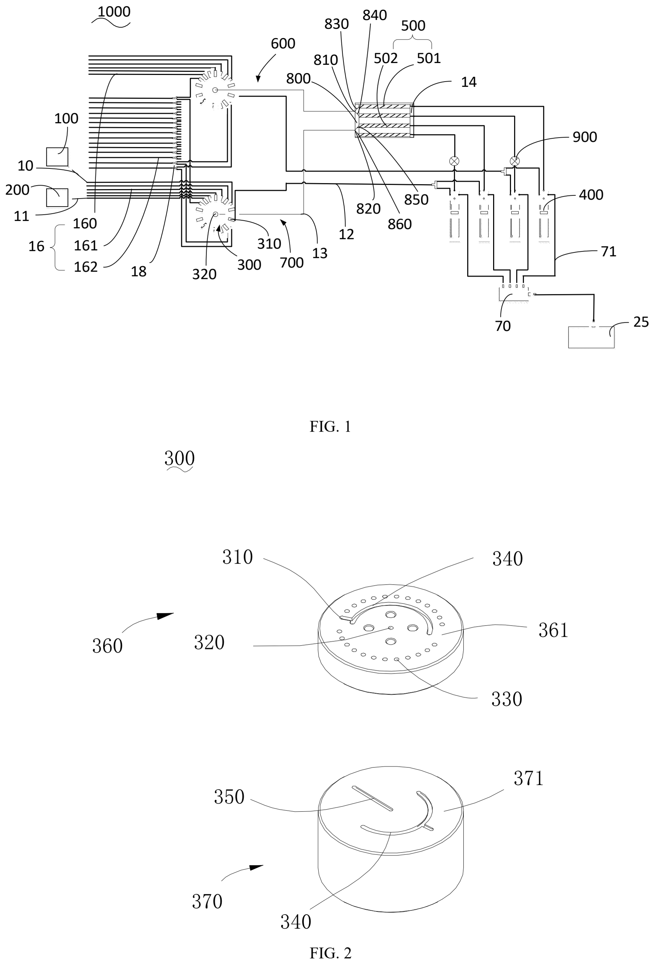

Referring to FIG. 1, a fluidic system 1000 according to the embodiments of the present application includes a plurality of reservoirs, a rotary valve 300, and a pump assembly 400. One of the reservoirs is capable of being in communication with the rotary valve 300 through a first flow path 10, the remaining reservoirs are capable of being in communication with the rotary valve 300 through a second flow path 11, and the rotary valve is connected to a reaction device 500 through a third flow path 13. The pump assembly 400 is in fluid connection with the rotary valve 300 through a fourth flow path 12. The fluidic system 1000 is selectively in a first state or a second state. When the fluidic system 1000 is in the first state, one of the reservoirs is in communication with the pump assembly 400 through the first flow path 10, the rotary valve 300, and the fourth flow path 12, and the pump assembly 400 is configured to drive the liquid in one of the reservoirs to enter the fourth flow path 12. When the fluidic system 1000 is in the second state, at least one remaining reservoir is in communication with the reaction device 500 through the second flow path 11, the rotary valve 300, and the third flow path 13, and the pump assembly 400 is configured to drive the liquid in the at least one remaining reservoir to enter the reaction device 500.

In the fluidic system 1000 according to the embodiments of the present application, the fluidic system 1000 is configured to be able to switch between the first state and the second state, with the liquid in the fluidic system 1000 entering the fourth flow path 12 or the reaction device 500 in different states. When the reservoirs store different liquids, the fluidic system 1000 may exhibit different functions. Further, when the reservoirs store a cleaning solution and a sequencing reagent, respectively, the fluidic system 1000 can exhibit both the cleaning function and sequencing function, such that the fluidic system 1000 can be cleaned in time after the completion of sequencing, and there is no sequencing reagent remaining in the fluidic system 1000, thereby ensuring the use performance of the instrument.

Specifically, the reservoirs include a first reservoir 100 and a second reservoir 200 that are independent of each other. The first reservoir 100 is in communication with the rotary valve 300 through the first flow path 10, and the second reservoir 200 is in communication with the rotary valve 300 through the second flow path 11.

The first reservoir 100 and the second reservoir 200 may be containers for accommodating liquid. The first reservoir 100 stores a first solution, and the second reservoir 200 stores a second solution. Structures and shapes of the first reservoir 100 and the second reservoir 200 are not limited, as long as the two reservoirs are independently configured. For example, the first reservoir 100 and the second reservoir 200 are two independent storage boxes, or the first reservoir 100 and the second reservoir 200 are two independent storage chambers in the same storage box.

Optionally, the liquid accommodated in the first reservoir 100 may include a cleaning solution. The liquid accommodated in the second reservoir 200 may include a sequencing reagent. The "sequencing reagent" refers to a reagent required during the detection and analysis of a nucleic acid under test, such as a reagent required in a sequencing reaction. The sequencing reagent includes an imaging reagent, a cleavage reagent, and the like. The "cleaning solution" refers to a liquid used to remove or replace another liquid or substance from the previous reaction system. When the fluidic system 1000 is in the first state, the pump assembly 400 drives the cleaning solution in the first reservoir 100 to enter the fourth flow path 12 through the rotary valve 300; when the fluidic system 1000 is in the second state, the pump assembly 400 drives the sequencing reagent in the second reservoir 200 to enter the reaction device 500 via the rotary valve 300.

The first reservoir 100 and the second reservoir 200 may both include a plurality of reagent tubes, and the plurality of reagent tubes are configured to accommodate the same or different liquids. The fluidic system 1000 according to the embodiments of the present application can achieve the transportation of liquid. The liquid may be of various types, including a reaction liquid, a biological sample liquid, a buffer, a cleaning solution, and the like, as well as reagents for different reactions or different steps of the same reaction. The fluidic system 1000 can transport the biological sample liquid into the reaction device 500 for corresponding reactions, such as a hybridization reaction and a sequencing reaction.

The biological sample liquid is a liquid containing a nucleic acid under test. The fluidic system 1000 transports the biological sample liquid into the fluid channel of the reaction device 500, such that the nucleic acid under test is complementarily paired with at least a portion of a probe, i.e., performing a hybridization reaction, so as to enable the immobilization or attachment of the nucleic acid under test on the surface of the fluid channel, thereby facilitating the subsequent detection and analysis of the nucleic acid under test, such as a sequencing reaction.

The reaction device 500 is a sandwich-like structure with upper, middle, and lower layers or a structure with upper and lower layers. The upper layer is a transparent glass layer, the middle layer or the lower layer is a transparent or opaque substrate layer, and the middle layer or the lower layer is provided with fluid channels arranged in an array. The fluid channels can accommodate liquid and provide physical space for reaction. The upper surface (the lower surface of the upper glass layer) or the lower surface (the upper surface of the middle layer or lower layer) of the fluid channel is provided with a probe (oligonucleotide). There may be a plurality of rotary valves 300, and the plurality of rotary valves 300 may integrate the functions of a plurality of three-way valves, achieving functions such as flow splitting for the fluidic system 1000 with a significantly reduced quantity by a factor of several times. This results in reduced cost, smaller size, decreased reagent consumption, improved reliability, and easier maintenance, repair, and control of the fluidic system 1000. The rotary valve 300 may be in communication with the first reservoir 100 through the first flow path 10, and the liquid in the first reservoir 100 may flow into the rotary valve 300 through the first flow path 10. The rotary valve 300 may be in communication with the second reservoir 200 through the second flow path 11, and the liquid in the second reservoir 200 may flow into the rotary valve 300 through the second flow path 11.

Referring to FIG. 1, in some embodiments, the reaction device 500 may include a first unit 501 and a second unit 502, and the number of first units 501 and second units 502 is not limited in the embodiments of the present application. Each of the first unit 501 and the second unit 502 includes at least one fluid channel. The number of fluid channels in the two units may be designed to be the same or different. In this embodiment, the first unit 501 and the second unit 502 both include two fluid channels.

The fluidic system 1000 can transport the same or different biological sample liquids into the fluid channels of the first unit 501 and the second unit 502 of the reaction device 500, so as to achieve detection and analysis of the same or different nucleic acids under test. In this way, the fluidic system 1000, including the reaction device 500, is beneficial for improving the sequencing throughput and/or achieving simultaneous detection of a plurality of samples. Such a configuration also avoids cross-contamination during the liquid supply of the fluidic system 1000 to the first unit 501 and the second unit 502.

Referring to FIG. 1, the fluidic system 1000 according to the embodiments of the present application is designed to include a first fluidic unit 600 and a second fluidic unit 700. The first fluidic unit 600 and the second fluidic unit 700 are independent of and in parallel with each other, so as to achieve liquid supply to the first unit 501 and the second unit 502, respectively. This is beneficial for the first unit 501 and the second unit 502 to perform different reactions or different steps of the same reaction, so as to achieve detection and analysis of the same or different nucleic acids under test.

Specifically, the first fluidic unit 600 and the second fluidic unit 700 both include a reagent switching system and a power system.

The reagent switching system is arranged upstream of the reaction device 500. Specifically, the reagent switching system includes a rotary valve 300 and a first manifold block 800. The rotary valve 300 can control different liquids to sequentially pass through the first manifold block 800 to enter the fluid channel of the reaction device 500. The first manifold block 800 includes a first liquid inlet 810, a second liquid inlet 820, a first liquid outlet 830, a second liquid outlet 840, a third liquid outlet 850, and a fourth liquid outlet 860.

The first liquid inlet 810 is separately in communication with the first liquid outlet 830 and the second liquid outlet 840, and the second liquid inlet 820 is separately in communication with the third liquid outlet 850 and the fourth liquid outlet 860. The liquid transported by the first fluidic unit 600 passes through the rotary valve 300 of the first fluidic unit 600, flows into the first manifold block 800 via the first liquid inlet 810, and is then split into two. Subsequently, the liquid flows out from the first liquid outlet 830 and the second liquid outlet 840 in a splitting manner.

The first liquid outlet 830 and the second liquid outlet 840 are respectively in communication with two fluid channels of the first unit 501 on the reaction device 500 in a one-to-one correspondence. The liquid transported by the first fluidic unit 600 flows out from the first liquid outlet 830 and the second liquid outlet 840 and then flows into the two fluid channels of the first unit 501, thereby achieving a separate liquid supply of the first fluidic unit 600 to the first unit 501 on the reaction device 500. The liquid transported by the second fluidic unit 700 passes through the rotary valve 300 of the second fluidic unit 700, flows into the first manifold block 800 via the second liquid inlet 820, and is then split into two. Subsequently, the liquid flows out from the third liquid outlet 850 and the fourth liquid outlet 860 in a splitting manner. The third liquid outlet 850 and the fourth liquid outlet 860 are respectively in communication with two fluid channels of the second unit 502 on the reaction device 500 in a one-to-one correspondence. The liquid transported by the second fluidic unit 700 flows out from the third liquid outlet 850 and the fourth liquid outlet 860 and then flows into the two fluid channels of the second unit 502, thereby achieving a separate liquid supply of the second fluidic unit 700 to the second unit 502 on the reaction device 500.

The first fluidic unit 600 and the second fluidic unit 700 independently and in parallel supply liquids to the first unit 501 and the second unit 502 of the reaction device 500. This is beneficial for the first unit 501 and the second unit 502 to perform different reactions or different steps of the same reaction, thereby achieving detection and analysis of the same or different nucleic acids under test. This is beneficial for improving the sequencing throughput and/or achieving simultaneous detection of a plurality of samples. It should be noted that the number of rotary valves 300 and first manifold blocks 800 may both be plural. The illustration is merely a schematic example and should not be construed as a limitation on the rotary valve 300 and the first manifold block 800 in the embodiments of the present application.

In some embodiments, the first manifold block 800 may be designed separately from the reaction device 500.

In some embodiments, the first manifold block 800 may be integrated with the reaction device 500 on the reaction device 500.

Referring to FIG. 1, in some embodiments, the power system provides power for the fluidic system 1000, such that the liquid in the fluidic system 1000 is capable of flowing.

Specifically, the power system may include a pump assembly 400, a pressure sensor 900, and a second manifold block 14. The pump assembly 400 is arranged downstream of the reaction device 500 and may provide negative pressure for the liquid to pass through the rotary valve 300 and flow into the fluid channel. Optionally, the number of pump assemblies 400 is the same as the number of fluid channels of the reaction device 500, such that the pump assemblies 400 can independently control the liquid in each fluid channel in the reaction device 500, which is beneficial for finely controlling the flow rate and/or flow velocity of the fluid in each fluid channel.

In addition, the pump assembly 400 is arranged downstream of the reaction device 500 and provides negative pressure. This can prevent liquid crossover or liquid leakage within the fluid channels inside the reaction device 500. The pressure sensor 900 is arranged on a conduit connected between the reaction device 500 and the pump assembly 400. The pressure sensor 900 can monitor the pressure of the fluidic system 1000 and give an alarm when the pressure is abnormal. The second manifold block 14 can be configured to combine the liquids flowing out of the reaction device 500, or in other words, liquids generated during sequencing. The liquid generated during sequencing may be a liquid generated after the reaction of the above sequencing reagent or the above sequencing reagent after use, or may be other liquids that maintain the reliability of the sequencing process, such as condensed water used to cool the sequencing environment.

The number of pump assemblies 400, pressure sensors 900, and second manifold blocks 14 may all be plural. The illustration is merely a schematic example and should not be construed as a limitation on the pump assembly 400, the pressure sensor 900, and the second manifold block 14 in the embodiments of the present application.

The switching between the first state and the second state of the fluidic system 1000 can be achieved by means of the pump assembly 400 and/or the rotary valve 300. For example, when the fluidic system 1000 is in the first state, the pump assembly 400 can drive the cleaning solution in the first reservoir 100 to enter the first flow path 10, the rotary valve 300, and the fourth flow path 12. Herein, the "cleaning" refers to introducing a liquid to remove or replace another liquid or substance in the previous reaction system, which generally does not involve substantial processing and/or biochemical reactions.

When the fluidic system 1000 is in the second state, the pump assembly 400 can drive the sequencing reagent in the second reservoir 200 to enter the reaction device 500 through the second flow path 11, the rotary valve 300, and the third flow path 13 to perform a sequencing reaction.

Referring to FIGs. 1 and 2, in some embodiments, the rotary valve 300 is provided with a first common port 310, a second common port 320, a plurality of connection ports 330, a first communication groove 340, and a second communication groove 350. The first common port 310 is connected to one end of the fourth flow path 12, and the other end of the fourth flow path 12 is connected to the pump assembly 400; the second common port 320 is connected to one end of the third flow path 13, and the other end of the third flow path 13 is connected to the reaction device 500. In the case where the rotary valve 300 is at a first valve position, the first common port 310 is in communication with the first flow path 10 via at least one of the connection ports 330 and the first communication groove 340; in the case where the rotary valve 300 is at a second valve position, the second common port 320 is in communication with the second flow path 11 via the remaining connection ports 330 and the second communication groove 350.

In this way, by configuring the rotary valve 300 to be able to switch between the first valve position and the second valve position, the rotary valve 300, when at different positions, can enable the first common port 310 and at least one of the connection ports 330 to be in communication with the first flow path 10 via the first communication groove 340, thereby achieving liquid supply of the first reservoir 100 to the fourth flow path 12. The second common port 320 and the remaining connection ports 330 are in communication with the second flow path 11 via the second communication groove 350, thereby achieving liquid supply of the second reservoir 200 to the third flow path 13. Such a configuration enables the rotary valve 300 to integrate the function of the three-way valve and have two common ports, simplifying the fluidic system 1000 and reducing the reagent consumption.

The communication between the first common port 310 and the connection port 330 may be achieved via the first communication groove 340. In the illustrated embodiment of the present application, a single first common port 310 is shown. The first common port 310 may serve as a liquid inlet or outlet of the rotary valve 300.

In other words, the liquid may enter or exit the rotary valve 300 from the first common port 310, thereby achieving flow splitting or flow confluence. Here, the first common port 310 serves as the liquid inlet of the rotary valve 300, and liquid may enter the rotary valve 300 from the first common port 310. The shape of the first common port 310 may be a regular shape such as circular or polygonal, or may be an irregular shape.

Similarly, the communication between the second common port 320 and the connection port 330 may be achieved via the second communication groove 350. In the illustrated embodiment of the present application, a single second common port 320 is shown. The second common port 320 may serve as a liquid inlet or outlet of the rotary valve 300. In other words, the liquid may enter or exit the rotary valve 300 from the second common port 320, thereby achieving flow splitting or flow confluence. Here, the second common port 320 serves as the liquid inlet of the rotary valve 300, and liquid may enter the rotary valve 300 from the second common port 320. The shape of the second common port 320 may be a regular shape such as circular or polygonal, or may be an irregular shape. In the embodiments of the present application, to facilitate the formation and manufacturing of the second common port 320 and/or the connection thereof with a common tube, the second common port 320 is circular in shape.

To facilitate the arrangement of the positions of the connection ports 330, the included angle between the first communication groove 340 and the second communication groove 350 may be set to 173.5°, i.e., the included angle between the extension line of the first communication groove 340 passing through the axis of the rotary valve 300 and the extension line of the second communication groove 350 passing through the axis of the rotary valve 300. The size of the included angle between the first communication groove 340 and the second communication groove 350 is not limited in the embodiments of the present application. Further, through the adjustment of the angle and the shape between the first communication groove 340 and the second communication groove 350, the positions of the first common port 310 and the second common port 320 on the rotary valve 300 can be adjusted.

Only one of the first common port 310 and the second common port 320 can be in communication with the connection port 330, and in this case, the other port is in a blocked state. In other words, when the first common port 310 is in communication with the connection port 330 via the first communication groove 340, the second common port 320 is in the blocked state (i.e., liquid cannot flow into the second common port 320); when the second common port 320 is in communication with the connection port 330 via the second communication groove 350, the first common port 310 is in the blocked state (i.e., liquid cannot flow into the first common port 310).

When the rotary valve 300 is rotated to the first valve position, the first common port 310 is in communication with the plurality of connection ports 330 via the first communication groove 340, and in this case, liquid enters the rotary valve 300 from the first common port 310 and flows out through the plurality of connection ports 330. When the rotary valve 300 is rotated to the second valve position, the second common port 320 is in communication with the plurality of connection ports 330 via the second communication groove 350, and in this case, liquid enters the rotary valve 300 from the second common port 320 and flows out through the plurality of connection ports 330.

Referring to FIGs. 1 and 2, in some embodiments, the rotary valve 300 includes a stator 360 and a rotor 370 arranged opposite to the stator 360. The stator 360 is provided with the first common port 310, the second common port 320, the plurality of connection ports 330, and at least a portion of the first communication groove 340, and the rotor 370 is provided with at least a portion of the second communication groove 350.

In this way, during the rotation of the rotor 370, the first communication groove 340 and the second communication groove can be in communication with the first common port 310 and the second common port 320, such that liquid can enter the rotary valve 300 from the stator 360 and flow out of the rotary valve 300 from the stator 360.

The rotor 370 and the stator 360 are coaxially arranged, or in other words, the central axis of the rotor 370 and the central axis of the stator 360 coincide. The second common port 320 may be arranged on the central axis. It can be understood that the rotor 370 can rotate relative to the stator 360. Further, the rotor 370 rotates relative to the stator 360 between the first valve position and the second valve position.

It should be noted that the rotary valve 300 rotates between the first valve position and the second valve position, meaning that it is the rotor 370 of the rotary valve 300 itself that rotates, rather than the entire rotary valve 300 rotating.

That the stator 360 is provided with at least a portion of the first communication groove 340 means that part or all of the structure of the first communication groove 340 may be provided on the stator 360. Similarly, that the rotor 370 is provided with at least a portion of the second communication groove 350 means that part or all of the structure of the second communication groove 350 may be provided on the rotor 370. It can be understood that the first communication groove 340 may consist of different portions provided on the stator 360 and the rotor 370, and the second communication groove 350 may consist of different portions provided on the stator 360 and the rotor 370.

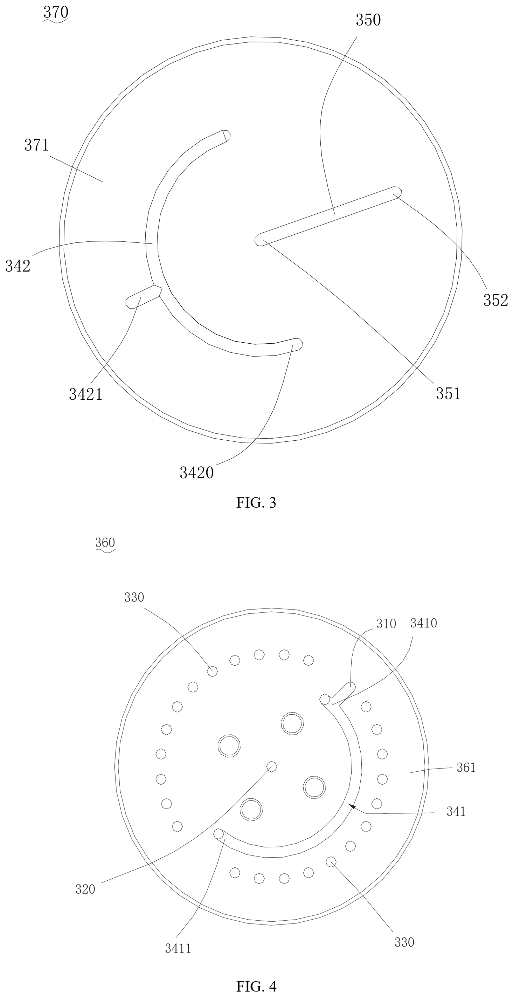

Referring to FIGs. 2, 3, and 4, in some embodiments, the first communication groove 340 includes a first groove 341 and a second groove 342 that can communicate with each other, and the first groove 341 and the second groove 342 are provided on the stator 360 and the rotor 370, respectively.

In this way, during the rotation of the rotor 370, the second groove 342 can move to a position where the second groove communicates with the first groove 341, such that liquid may flow from the first groove 341 to the second groove 342.

Specifically, the first groove 341 may be recessed into and formed on the end surface of the stator 360 in the axial direction of the stator 360, and the second groove 342 may be provided on the rotor 370 with the opening of the second groove 342 facing the stator 360. The first groove 341 may be in communication with or separated apart from the second groove 342 during the rotation of the rotor 370. For example, further, when the rotor 370 rotates and positions the rotary valve 300 in the first valve position, the first groove 341 is in communication with the second groove 342.

Referring to FIGs. 2, 3, and 4, in some embodiments, the first groove 341 is provided with two ends, with one end of the first groove 341 being in communication with the first common port 310, and the other end being in communication with the second groove 342; the second groove 342 is provided with two ends, with one end of the second groove 342 being in communication with the first groove 341 and the other end being in selective communication with one connection port 330.

In this way, the first common port 310 can be in communication with one connection port 330 via the first groove 341 and the second groove 342.

Specifically, for ease of function implementation, both the first groove 341 and the second groove 342 may be arc-like grooves, or the first groove 341 and the second groove 342 may be in other shapes. Specific shapes and structures of the first groove 341 and the second groove 342 are not limited in the embodiments of the present application. The first groove 341 may include a first end 3410 of the first groove 341 and a second end 3411 of the first groove 341. The first end 3410 of the first groove 341 may be arranged at a position near the first common port 310, and the second end 3411 of the first groove 341 may be arranged at a position away from the first common port 310.

Similarly, the second groove 342 may include a first end 3420 of the second groove 342 and a second end 3421 of the second groove 342. The second end 3421 of the second groove 342 is an end that is on the arc of the second groove 342 and in communication with one connection port 330. The second end 3421 of the second groove 342 may be arranged perpendicular to the arc of the second groove 342.

When the second common port 320 is in the blocked state, liquid flows into the rotary valve 300 through the first common port 310 and flows through the first end 3410 of the first groove 341 to the second end 3411 of the first groove 341 along the arc of the first groove 341. Subsequently, the liquid flows from the second end 3411 of the first groove 341 to the first end 3420 of the second groove 342, then flows from the first end 3420 of the second groove 342 to the second end 3421 of the second groove 342 along the arc of the second groove 342, and flows from the second end 3421 of the second groove 342 to one connection port 330. Finally, the liquid flows out of the stator 360.

It should be noted that during the rotation of the rotor 370, the second end 3421 of the second groove 342 may be in selective communication with one connection port 330. That is, when the rotor 370 rotates to different positions, the second end 3421 of the second groove 342 is separately connected to one corresponding connection port 330.

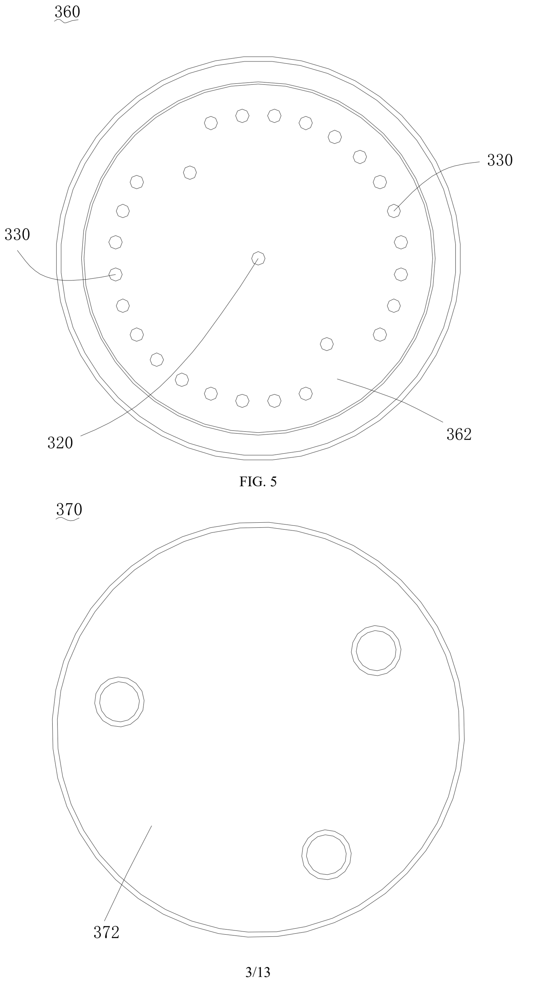

Referring to FIGs. 2 to 6, in some embodiments, the stator 360 includes a first end surface 361 and a second end surface 362 that are opposite to each other, and the rotor 370 includes a third end surface 371 and a fourth end surface 372 that are opposite to each other. The second end surface 362 and the third end surface 371 are in close contact; the first groove 341 is formed on the first end surface 361, and the second groove 342 is formed on the third end surface 371.

In this way, the close contact between the second end surface 362 and the third end surface 371 can prevent liquid leakage. The first groove 341 and the second groove 342 are formed on the first end surface 361 and the third end surface 371, respectively, making it easier to manufacture and form the first communication groove 340, which can reduce the manufacturing costs of the stator 360 and the rotor 370.

Specifically, the first end surface 361 is an end surface on which liquid flows into the stator 360 for the first time, and the first stator 360 and the rotor 370 are in close contact through the second end surface 362 and the third end surface 371, such that the liquid can flow only along the first groove 341 and the second groove 342 after entering the stator 360 and rotor 370 portions of the rotary valve 300. The first groove 341 is formed on the first end surface 361, and the opening of the first groove 341 is provided facing away from the third end surface 371 of the rotor 370. The second groove 342 is formed on the third end surface 371, and the opening of the second groove 342 faces the second end surface 362 of the stator 360.

Referring to FIGs. 2, 3, and 5, in some embodiments, the second communication groove 350 is provided with two ends, with one end of the second communication groove 350 being in communication with the second common port 320 and the other end being in selective communication with one connection port 330.

In this way, the second communication groove 350 enables the second common port 320 to be in communication with one connection port 330.

Specifically, the second communication groove 350 may be a linear groove, and the second communication groove 350 may be formed on the third end surface 371. When the first common port 310 is in the blocked state, liquid flows into the rotary valve 300 through the second common port 320. Further, the liquid flows into the first end 351 of the second communication groove 350 through the second common port 320, then flows through the first end 351 of the second communication groove 350 into the second end 352 of the second communication groove 350 along the straight line of the second communication groove 350, and finally flows into one connection port 330.

It should be noted that during the rotation of the rotor 370, the second end 352 of the second communication groove 350 can be in selective communication with one connection port 330. That is, when the rotor 370 rotates to different positions, the second end 352 of the second communication groove 350 is separately connected to one corresponding connection port 330.

Referring to FIGs. 2 to 6, in some embodiments, the stator 360 includes a first end surface 361 and a second end surface 362 that are opposite to each other, and the rotor 370 includes a third end surface 371 and a fourth end surface 372 that are opposite to each other. The second end surface 362 and the third end surface 371 are in close contact, and the second communication groove 350 is formed on the third end surface 371. In this way, the close contact between the second end surface 362 and the third end surface 371 can prevent liquid leakage. The second communication groove 350 is formed on the third end surface 371, which can reduce the manufacturing costs of the stator 360 and the rotor 370.

Specifically, the second communication groove 350 is recessed into and formed on the third end surface 371 of the rotor 370, and the opening of the second communication groove 350 faces the second end surface 362 in the axial direction of the stator 360.

Referring to FIG. 1, in some embodiments, when the fluidic system 1000 is in the first state, the pump assembly 400 further drives the liquid in the fourth flow path 12 to flow back to the second flow path 11 to clean the second flow path 11. Specifically, the pump assembly 400 drives the cleaning solution in the fourth flow path 12 to flow back to the second flow path 11 to clean the second flow path 11.

In this way, the reagent for sequencing remaining in the second flow path 11 can be cleaned.

Specifically, when the fluidic system 1000 is in the first state, the pump assembly 400 can generate negative pressure to drive the liquid in the first reservoir 100 to flow to the fourth flow path 12 through the rotary valve 300. Then, the pump assembly 400 can generate positive pressure to drive the liquid in the fourth flow path 12 to flow back to the rotary valve 300, and the liquid flows through the second common port 320 of the rotary valve 300 into the second flow path 11 and cleans the second flow path 11.

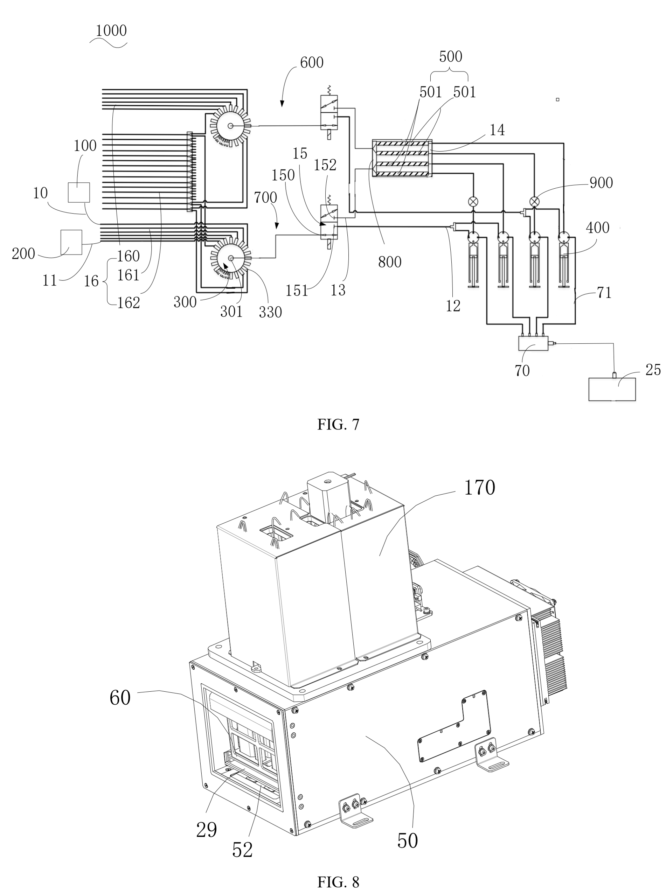

Referring to FIG. 7, in some embodiments, the fluidic system 1000 includes a multi-way valve 15 arranged between the rotary valve 300 and the reaction device 500. The multi-way valve 15 changes the path thereof to enable the fluidic system 1000 to be in the first state or the second state.

In this way, the fluidic system 1000 can switch between the first state and the second state by means of the multi-way valve 15, thus enabling the fluidic system 1000 to achieve both the cleaning and sequencing functions.

Specifically, in the fluidic system 1000 provided with the multi-way valve 15, the liquid outlet of the rotary valve 300 is the second common port 320. The number of multi-way valves 15 may be in a one-to-one correspondence with the number of rotary valves 300, such that liquids passing through different rotary valves 300 are independently controlled. The multi-way valve 15 can be separately connected to the fourth flow path 12 and the third flow path 13, and the multi-way valve 15 changes the path thereof to enable the fluidic system 1000 to be in the first state or the second state. For example, the multi-way valve 15 can close the passage between itself and the fourth flow path 12 to enable the fluidic system 1000 to be in the second state. For another example, the multi-way valve 15 can close the passage between itself and the third flow path 13 to enable the fluidic system 1000 to be in the first state.

Referring to FIG. 7, in some embodiments, the multi-way valve 15 includes a first through port 150, a second through port 151, and a third through port 152. The first through port 150 is in selective communication with the second through port 151 or the third through port 152, and the first through port 150 is in communication with the rotary valve 300. The second through port 151 is connected to one end of the fourth flow path 12, and the other end of the fourth flow path 12 is connected to the pump assembly 400. The third through port 152 is connected to one end of the third flow path 13, and the other end of the third flow path 13 is connected to the reaction device 500. When the first through port 150 is in communication with the second through port 151, the fluidic system 1000 is in the first state, and when the first through port 150 is in communication with the third through port 152, the fluidic system 1000 is in the second state.

In this way, through the separate communication of the first through port 150 with the second through port 151 and the third through port 152, the fluidic system 1000 can be in the first state and the second state, respectively. Therefore, the fluidic system 1000 can achieve both the cleaning and sequencing functions. Specifically, the multi-way valve 15 may be a solenoid valve, and the solenoid valve may include an electromagnet. The solenoid valve controls the communication of the first through port 150 with the second through port 151 and the third through port 152 by controlling the energization and de-energization of the electromagnet. When the first through port 150 is in communication with the second through port 151, the first reservoir 100 and the rotary valve 300 are in communication with the pump assembly 400 through the fourth flow path 12. In this case, the fluidic system 1000 is in the first state, and the cleaning solution in the first reservoir 100 can enter the first flow path 10, the rotary valve 300, and the fourth flow path 12. When the first through port 150 is in communication with the third through port 152, the second reservoir 200 and the rotary valve 300 are in communication with the reaction device 500 through the third flow path 13, and the sequencing reagent in the second reservoir 200 can enter the reaction device 500 to perform a sequencing reaction.

Referring to FIG. 7, in some embodiments, the rotary valve 300 includes a common port 301 and a plurality of connection ports 330. The common port 301 is in selective communication with at least one of the connection ports 330, the common port 301 is in communication with the first through port 150, and the first flow path 10 and the second flow path 11 are connected to the corresponding connection ports 330, respectively. When the rotary valve 300 is at the first valve position, the common port 301 is in communication with the first flow path 10 via at least one of the connection ports 330; when the rotary valve 300 is at the second valve position, the common port 301 is in communication with the second flow path 11 via the remaining connection ports 330.

In this way, when the rotary valve 300 is at different positions, the common port 301 can be separately in communication with the first flow path 10 and the second flow path 11 via different connection ports 330, thereby simplifying the fluidic system 1000.

Specifically, through the rotation of the rotor 370 relative to the stator 360, the common port 301 can be connected to different connection ports 330, thereby enabling the connection thereof to the first flow path 10 or the second flow path 11.

It should be noted that for other undescribed parts of the fluidic system 1000 in the embodiment shown in FIG. 7, reference may be made to the same or similar parts of the fluidic system 1000 in the embodiment shown in FIG. 1, and details are not described herein again.

Referring to FIGs. 1 and 7 to 10, in some embodiments, the fluidic system 1000 includes a first reagent needle 16 and a first driving mechanism 17. The first reagent needle 16 is connected to the first flow path 10 and/or the second flow path 11, and the first driving mechanism 17 is configured to drive the first reagent needle 16 to move in the length direction of the first reagent needle 16, such that the first reagent needle 16 extends into the first reservoir 100 and/or the second reservoir 200.

In this way, under the driving of the first driving mechanism 17, the first reagent needle 16 can extend into the first reservoir 100 and/or the second reservoir 200, so as to aspirate the liquid in the first reservoir 100 and/or the second reservoir 200.

It should be noted that in the case where the number of first reagent needles 16 is one, the first reagent needle 16 can extend into the first reservoir 100 or the second reservoir 200 each time; in the case where the number of first reagent needles 16 is plural, the plurality of first reagent needles 16 can extend separately into the first reservoir 100 and the second reservoir 200 each time, or all the plurality of first reagent needles 16 can extend into one of the first reservoir 100 and the second reservoir 200 each time.

Specifically, the first reagent needle 16 can be configured to aspirate a reaction liquid, a biological sample liquid, or other liquids. After a liquid enters the first reagent needle 16, the liquid can flow into the reaction device 500 through the rotary valve 300 to perform a corresponding reaction under the negative pressure provided by the pump assembly 400; the rotary valve 300 can select different liquids to sequentially flow into the reaction device 500 to complete corresponding reactions.

The first reagent needle 16 includes a first type of reagent needle 160, a second type of reagent needle 161, and a third type of reagent needle 162. The number of first reagent needles 160, second reagent needles 161, and third reagent needles 162 is not limited, and the three types of reagent needles are configured to aspirate corresponding liquids.

The first type of reagent needle 160 is in communication with the rotary valve 300 of the first fluidic unit 600 through a conduit, enabling the liquid aspirated by the first type of reagent needle 160 to enter the first unit 501 of the reaction device 500 through the rotary valve 300 of the first fluidic unit 600. The second type of reagent needle 161 is in communication with the rotary valve 300 of the second fluidic unit 700 through a conduit, enabling the liquid aspirated by the second type of reagent needle 161 to enter the second unit 502 of the reaction device 500 through the rotary valve 300 of the second fluidic unit 700. The third type of reagent needle 162 is separately in communication with the rotary valves 300 of the first fluidic unit 600 and the second fluidic unit 700 through the third manifold block 18 (as shown in FIG. 1), enabling the liquid aspirated by the third type of reagent needle 162 to simultaneously enter the first unit 501 and the second unit 502 of the reaction device 500 through the rotary valves 300 of the first fluidic unit 600 and the second fluidic unit 700, respectively.

The third manifold block 18 is provided with a plurality of "Y"-shaped channels arranged side by side in the length direction, and the "Y"-shaped channels may split the liquid flowing into the third manifold block 18 into two. The "Y"-shaped channel includes one liquid inlet and two liquid outlets. The liquid inlet of the "Y"-shaped channel is in communication with the third type of reagent needle 162 through a conduit, and the two liquid outlets of the "Y"-shaped channel are in communication with the rotary valve 300 of the first fluidic unit 600 and the rotary valve 300 of the second fluidic unit 700 through conduits, respectively.

The liquid aspirated by the third type of reagent needle 162, after being split into two by the third manifold block 18, may flow into the first unit 501 and the second unit 502 of the reaction device 500 through the rotary valve 300 of the first fluidic unit 600 and the rotary valve 300 of the second fluidic unit 700, respectively, thereby enabling the fluidic system 1000 to provide the first unit 501 and the second unit 502 of the reaction device 500 with the same type of liquid simultaneously, and thus improving the liquid supply efficiency.

Any one of the first type of reagent needles 160 and any one of the second type of reagent needles 161 may form a double-needle unit inserted into the same reservoir, so as to aspirate the sequencing reagent stored in the reservoir simultaneously or in a time-sharing manner, thereby supplying liquids to the first fluidic unit 600 and the second fluidic unit 700 separately. This enables the first unit 501 and the second unit 502 to perform the same reaction, different reactions, or different steps of the same reaction and meanwhile reduces the number of reservoirs for storing the same liquid, thereby enabling a more compact structure and a smaller volume of the fluidic system 1000.

The first reservoir 100 and/or the second reservoir 200 are provided with a plurality of liquid access ports 19 on the surface near the first reagent needle 16, and the first reagent needle 16 extends into the first reservoir 100 and/or the second reservoir 200 via the liquid access ports 19. The first reagent needle 16 can be configured to aspirate the liquids stored in the first reservoir 100 and the second reservoir 200. The first reagent needle 16 is in fluid connection with the first flow path 10 and/or the second flow path 11, and the first reagent needle 16 may be fixed onto the first driving mechanism 17 by means of a threaded connection or the like.

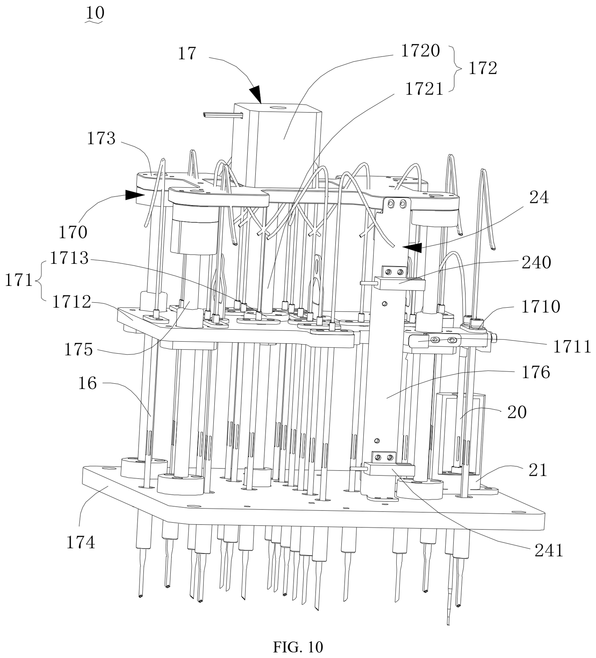

Referring to FIG. 10, in some embodiments, the first driving mechanism 17 includes a fixed frame 170, a movable member 171, and a first driving assembly 172. The movable member 171 is movably arranged on the fixed frame 170. The first driving assembly 172 is arranged on the fixed frame 170, and the first reagent needle 16 is mounted on the movable member 171. The first driving assembly 172 is configured to drive the movable member 171 to move relative to the fixed frame 170.

In this way, through the first driving assembly 172, the first reagent needle 16 mounted on the movable member 171 can move relative to the fixed frame 170.

Specifically, the fixed frame 170 may be arranged above the first reservoir 100 and the second reservoir 200, and the first reagent needle 16 may be fixed on the fixed frame 170. Further, the first reagent needle 16 may be fixed on the movable member 171. The first driving assembly 172 drives the movable member 171 to move relative to the fixed frame 170, thereby driving the first reagent needle 16 to move in the length direction of the first reagent needle 16.

Referring to FIG. 10, in some embodiments, the fixed frame 170 includes a first plate 173, a second plate 174, and connecting posts 175. The first plate 173 is spaced apart from the second plate 174, and the connecting posts 175 connect the first plate 173 and the second plate 174. The movable member 171 is movably arranged on the connecting posts 175 in a sleeving manner, and the first driving assembly 172 is mounted on the first plate 173 and the second plate 174.

In this way, the first driving assembly 172 can drive the movable member 171, which is arranged on the connecting posts 175 in a sleeving manner, to move, thereby driving the first reagent needle 16 fixedly connected to the movable member 171 to move in the length direction of the first reagent needle 16. Specifically, the first reagent needle 16 can be arranged passing through the first plate 173, the second plate 174, and the movable member 171 and fixedly connected to the movable member 171. The first plate 173, the movable member 171, and the second plate 174 may be arranged in an up, middle, or down configuration. The first plate 173 and the second plate 174 are connected to both ends of the connecting posts 175, respectively, and are fixedly connected to the connecting posts 175. The movable member 171 may move between the first plate 173 and the second plate 174 in the axial direction of the connecting posts 175, and the axial direction of the connecting posts 175 is parallel to the length direction of the first reagent needle 16.

Referring to FIG. 10, in some embodiments, the first driving assembly 172 includes a first motor 1720 and a first lead screw 1721 connected to the first motor 1720. The movable member 171 is arranged on the first lead screw 1721 in a sleeving manner, and the first motor 1720 drives the movable member 171 to move via the first lead screw 1721.

In this way, the first motor 1720 drives the movable member 171 via the first lead screw 1721, such that the movable member 171 achieves good position precision and motion repeatability.

Specifically, the first motor 1720 can be arranged on the first plate 173 by means of a fixed connection. One end of the first lead screw 1721 is connected to the first motor 1720 and arranged passing through the first plate 173, and the movable member 171 is arranged on the first lead screw 1721 in a sleeving manner. When the motor moves, the first lead screw 1721 is driven to move in the axial direction of the first lead screw 1721, thereby enabling the first lead screw 1721 to drive the movable member 171 to move, and further enabling the first reagent needle 16 fixed on the movable member 171 to move along with the movable member 171. The axial direction of the first lead screw 1721 is parallel to the length direction of the first reagent needle 16.

Specifically, the movable member 171 includes a movable plate 1712 arranged on the connecting posts 175 in a sleeving manner and a nut seat 1713 arranged on the first lead screw 1721 in a sleeving manner. The nut seat 1713 is in a threaded connection with the first lead screw 1721, and the nut seat 1713 is fixedly connected to the movable plate 1712. The first lead screw 1721 drives the nut seat 1713 to move through the threaded fit with the nut seat 1713. Since the nut seat 1713 is fixedly connected to the movable plate 1712, the nut seat 1713 drives the movable plate 1712 to move, thereby enabling the first reagent needle 16 fixed on the movable plate 1712 to move along with the movable plate 1712.

Referring to FIGs. 1, 7, and 10, in some embodiments, the fluidic system 1000 includes a second reagent needle 20 and a mounting base 21. The second reagent needle 20 is shorter than the first reagent needle 16. The mounting base 21 is mounted on the fixed frame 170. The second reagent needle 20 is connected to the first flow path 10 and/or the second flow path 11, and the second reagent needle 20 is mounted on the mounting base 21. The movable member 171, when moving toward the first reservoir 100 and/or the second reservoir 200, drives the second reagent needle 20 to extend into the first reservoir 100 and/or the second reservoir 200.

In this way, the fluidic system 1000 can be adapted to reagent needles of different sizes, thereby enabling high applicability of the fluidic system 1000. The second reagent needle 20 can be configured to aspirate a biological sample solution.

Specifically, the second reagent needle 20 is mounted on the second plate 174 of the fixed frame 170 by means of a bolt connection or the like, and one end of the second reagent needle 20 is in fluid connection with the first flow path 10 and/or the second flow path 11. The second reagent needle 20 is mounted on the mounting base 21 and arranged passing through the second plate 174, and the second reagent needle 20 is fixedly connected to the movable member 171. The movable member 171, when moving toward the first reservoir 100 and/or the second reservoir 200, can drive the second reagent needle 20 to extend into the first reservoir 100 and/or the second reservoir 200.

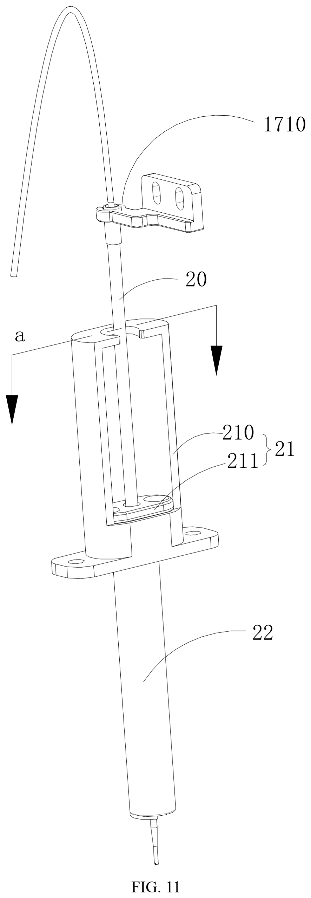

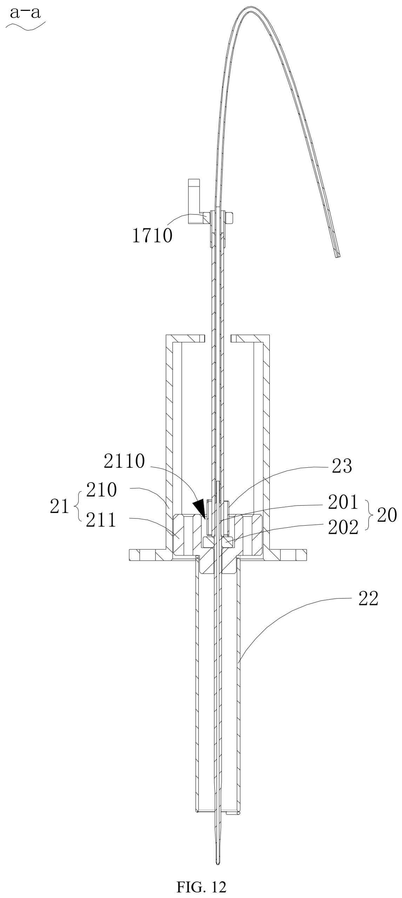

Referring to FIGs. 1, 7, and 10 to 12, in some embodiments, the mounting base 21 includes a guiding member 210 and a sliding member 211. The guiding member 210 is mounted on the fixed frame 170, and the sliding member 211 is movably arranged on the guiding member 210. The second reagent needle 20 is mounted on the sliding member 211, and the movable member 171, when moving toward the first reservoir 100 and/or the second reservoir 200, drives the sliding member 211 to move.

In this way, the movable member 171, when moving toward the first reservoir 100 and/or the second reservoir 200, can drive the sliding member 211 to move, thereby driving the second reagent needle 20 mounted on the sliding member 211 to move.

Specifically, the guiding member 210 may be fixedly mounted on the fixed frame 170 by means of a bolt connection or the like. The guiding member 210 may be provided with a slot. The slot extends in the axial direction of the guiding member 210. The sliding member 211 may be arranged in the slot, and the slot wall of the slot is configured to guide the sliding member 211, such that the sliding member 211 does not shake during movement.

Referring to FIGs. 1, 7, and 10 to 12, in some embodiments, a first elastic member 22 is arranged between the sliding member 211 and the fixed frame 170. The first elastic member 22 is compressed to store energy when the movable member 171 moves toward the first reservoir 100 and/or the second reservoir 200, and releases the stored energy after the force between the movable member 171 and the sliding member 211 is eliminated, so as to move the second reagent needle 20 outward from the first reservoir 100 and/or the second reservoir 200.

In this way, the first elastic member 22 can move the second reagent needle 20 outward from the first reservoir 100 and/or the second reservoir 200, thereby enabling the second reagent needle 20 to exit the first reservoir 100 and/or the second reservoir 200, and avoiding interference with the reservoir during the lateral movement of the second reagent needle 20 relative to the reservoir.

Specifically, the movable member 171 may include a pressing piece 1710. When the movable member 171 moves toward the first reservoir 100, the pressing piece 1710 can press against the sliding member 211, so as to move the sliding member 211 toward the first reservoir 100 and/or the second reservoir 200, thereby driving the second reagent needle 20 to move toward the first reservoir 100 and/or the second reservoir 200. When the second reagent needle 20 abuts against the bottom of the reservoir, the force between the movable member 171 and the sliding member 211 is eliminated, and the stored energy of the first elastic member 22 is released, such that the second reagent needle 20 can move outward from the first reservoir 100 and/or the second reservoir 200. When the force between the first elastic member 22 and the second reagent needle 20 is balanced, the movement of the second reagent needle 20 toward the first reservoir 100 and/or the second reservoir 200 is limited.

Referring to FIGs. 1, 7, and 10 to 12, in some embodiments, a second elastic member 23 is connected between the sliding member 211 and the second reagent needle 20. The second elastic member 23 applies a force in a first direction to the second reagent needle 20 so as to keep the second reagent needle 20 on the sliding member 211. When the second reagent needle 20 is subjected to a force in a second direction, the second elastic member 23 is compressed under the action of the second reagent needle 20. The first direction is a direction in which the second reagent needle 20 faces the first reservoir 100 and/or the second reservoir 200, and the second direction is opposite to the first direction.

In this way, the second elastic member 23 can keep the second reagent needle 20 on the sliding member 211 and offset the force generated when the second reagent needle 20 comes into contact with the bottom of the reservoir, thereby preventing the second reagent needle 20 from puncturing the reservoir or bending the second reagent needle 20.

Specifically, when the pressing piece 1710 presses against the sliding member 211, the second elastic member 23 applies a force to the second reagent needle 20 toward the first reservoir 100 and/or the second reservoir 200, such that the second reagent needle 20 moves along with the sliding member 211 in the first direction. When the second reagent needle 20 comes into contact with the bottom of the reservoir, the bottom of the reservoir provides an upward force to the second reagent needle 20. Under the combined action of this force and the downward force applied by the pressing piece 1710, the second elastic member 23 will be compressed in this case. In this way, the interaction force between the second reagent needle 20 and the reservoir is maintained within a certain range to prevent the second reagent needle 20 from puncturing the reservoir or bending the second reagent needle 20.

Referring to FIGs. 1, 7, and 10 to 12, in some embodiments, the sliding member 211 is provided with a mounting hole 2110, and the second reagent needle 20 includes a needle body 201 and a flange 202 arranged on the needle body 201. The needle body 201 passes through the sliding member 211, the flange 202 is arranged in the mounting hole 2110, and the second elastic member 23 abuts against the flange 202.

In this way, the second elastic member 23 applies a force to the second reagent needle 20 by abutting against the flange 202, such that the movement of the second reagent needle 20 away from the first reservoir 100 and/or the second reservoir 200 is limited.

Specifically, the mounting hole 2110 may be of a regular shape such as circular or square, or may be of an irregular shape. The needle body 201 may be configured to aspirate liquid from the first reservoir 100 and/or the second reservoir 200. The flange 202 may be arranged circumferentially around the needle body 201, and the radial dimension of the flange 202 is smaller than the aperture of the mounting hole 2110. When the sliding member 211 moves toward the first reservoir 100 and/or the second reservoir 200, the second elastic member 23 abuts against the flange 202, thereby driving the needle body 201 to move toward the first reservoir 100 and/or the second reservoir 200.

Referring to FIGs. 1, 7, and 10, in some embodiments, the fluidic system 1000 includes a first detection assembly 24 arranged on the fixed frame 170. The first detection assembly 24 is configured to detect the position of the movable member 171.

In this way, the position of the movable member 171 can be detected. The stroke of the movable member 171 is fixed, and the movable member 171 will not interfere with the first plate 173 or the second plate 174 during movement. In addition, by detecting the position of the movable member 171, the position where the reagent needle is inserted into the reservoir or exits the reservoir can be determined, thereby preventing the reagent needle from moving laterally relative to the reservoir when the reagent needle is inserted into the reservoir.

Specifically, the first detection assembly 24 may be fixedly connected to the fixed frame 170. In other words, the relative positions of the first detection assembly 24 and the fixed frame 170 remain unchanged. Referring to FIGs. 1, 7, and 10, in some embodiments, the first detection assembly 24 includes a first photoelectric switch 240 and a second photoelectric switch 241 spaced apart in the motion direction of the movable member 171. The first photoelectric switch 240 and the second photoelectric switch 241 are configured to detect the upper limit position and the lower limit position of the movable member 171, respectively.

In this way, compared with other detection assemblies, the photoelectric switch offers strong anti-interference capability and high detection reliability.

Specifically, the fixed frame 170 may include a support plate 176. The first photoelectric switch 240 and the second photoelectric switch 241 may be spaced apart on the support plate 176 in the motion direction of the movable member 171. A light shielding member 1711 is arranged on the movable member 171. The light shielding member 1711 can cooperate with the first photoelectric switch 240 or the second photoelectric switch 241 to detect the upper limit position and the lower limit position of the movable member 171.

For example, when the movable member 171 moves toward the first reservoir 100 and/or the second reservoir 200 such that the first reagent needle 16 and the second reagent needle 20 can aspirate liquid, and if the light shielding member 1711 blocks the second photoelectric switch 241, the movable member 171 moves to the lower limit position. For another example, when the movable member 171 moves away from the first reservoir 100 and/or the second reservoir 200, if the light shielding member 1711 blocks the first photoelectric switch 240, the movable member 171 moves to the upper limit position. The lower limit position is a position where the movable member 171 is closest to the first reservoir 100 and/or the second reservoir 200, and the upper limit position is a position where the movable member 171 is farthest from the first reservoir 100 and/or the second reservoir 200.

Referring to FIGs. 1, 7, and 13, in some embodiments, the fluidic system 1000 includes a liquid collector 25 configured to collect the liquid generated by the fluidic system 1000, and the liquid collector 25 is provided with a liquid inlet 250. The fluidic system 1000 further includes a cover assembly 26. The first driving mechanism 17 drives the cover assembly 26 to close and press against the liquid inlet 250 in the process of driving the first reagent needle 16 to extend into the first reservoir 100 and/or the second reservoir 200. Alternatively, the first driving mechanism 17 drives the cover assembly 26 to open the liquid inlet 250 in the process of driving the first reagent needle 16 to exit the first reservoir 100 and/or the second reservoir 200.

In this way, the process in which the cover assembly 26 closes the liquid inlet 250 is synchronized with the extension process of the first reagent needle 16 into the first reservoir 100 and/or the second reservoir 200, and the process in which the cover assembly 26 opens the liquid inlet 250 is synchronized with the exit process of the first reagent needle 16 from the first reservoir 100 and/or the second reservoir 200. This enables the cover assembly 26 to automatically close the liquid inlet 250 when the fluidic system 1000 generates liquid, and to open the liquid inlet 250 when the fluidic system 1000 does not generate liquid, thereby facilitating the use of the fluidic system 1000.

Specifically, the liquid generated by the fluidic system 1000 flows into the liquid collector 25 arranged downstream of the pump assembly 400 through a liquid discharge tube 71. A liquid manifold block 70 is further arranged upstream of the liquid collector 25. The liquid manifold block 70 is configured to converge the liquid generated in the fluidic system 1000 and input the liquid to the liquid collector 25. The first driving mechanism 17 can simultaneously drive the first reagent needle 16 and the cover assembly 26, thereby enabling linkage between the first reagent needle 16 and the cover assembly 26.

Referring to FIGs. 1, 7, and 13, in some embodiments, the first driving mechanism 17 includes a fixed frame 170, a movable member 171 movably arranged on the fixed frame 170, and a first driving assembly 172 arranged on the fixed frame 170. The first reagent needle 16 is mounted on the movable member 171. The first driving assembly 172 is configured to drive the movable member 171 to move relative to the fixed frame 170. During the movement of the movable member 171, the movable member 171 is capable of driving the cover assembly 26 to enable the cover assembly 26 to close and press against the liquid inlet 250 or open the liquid inlet 250.

In this way, the first reagent needle 16 is mounted on the movable member 171, and the movable member 171 drives the cover assembly 26 to move. This enables the first reagent needle 16 to be in linkage with the cover assembly 26, thereby simplifying the structure and control logic, and reducing the failure rate and cost of the first driving mechanism 17.

Specifically, the first reagent needle 16 may be fixedly connected to the movable member 171. The first driving assembly 172 can drive the movable member 171 to move toward or away from the first reservoir 100 and/or the second reservoir 200, thereby driving the first reagent needle 16 mounted on the movable member 171 to move toward or away from the first reservoir 100 and/or the second reservoir 200.

The first reagent needle 16 may be fixedly connected to the movable member 171. For example, the first reagent needle 16 may be arranged to pass through the movable member 171, and a metal plate may be interference-fitted in the circumferential direction of the first reagent needle 16. Both the metal plate and the movable member 171 may be provided with bolt holes, and the metal plate and the movable member 171 may be fixedly connected together by bolt connection, thereby enabling the movable member 171 to be indirectly connected to the first reagent needle 16. Since there may be a plurality of first reagent needles 16, arranging the movable member 171 to be indirectly connected to the first reagent needles 16 enables different first reagent needles 16 to be assembled and disassembled independently.

Referring to FIGs. 1, 7, and 13, in some embodiments, the cover assembly 26 includes a guide rail 260, a connecting frame 261, and a gland 262. The guide rail 260 is mounted on the fixed frame 170, the connecting frame 261 is movably arranged on the guide rail 260, and the gland 262 is arranged on the connecting frame 261. During the movement of the movable member 171, the movable member 171 is capable of driving the connecting frame 261 to enable the gland 262 to close and press against the liquid inlet 250 or open the liquid inlet 250.

In this way, by allowing the connecting frame 261 to drive the movable member 171, the gland 262 can close and press against the liquid inlet 250 or open the liquid inlet 250, thereby preventing the liquid generated by the fluidic system 1000 from leakage or evaporation, avoiding the formation of aerosols, and allowing the liquid collector 25 to be removed when no liquid is generated by the fluidic system 1000. Specifically, the guide rail 260 may be mounted on the fixed frame 170 by means of a bolt connection or the like. The connecting frame 261 can move on the guide rail 260, the gland 262 may be mounted on the connecting frame 261 by means of a bolt connection or the like, and the movement of the connecting frame 261 can drive the gland 262 to move. The connecting frame 261 is fixedly connected to the movable member 171 by means of a bolt connection or the like, such that the movement of the movable member 171 can drive the connecting frame 261 to move, so as to drive the gland 262 to move, thereby enabling the gland 262 to close and press against the liquid inlet 250 or open the liquid inlet 250.

Referring to FIGs. 1, 7, 14, and 15, in some embodiments, the connecting frame 261 is provided with a first limiting position 2610 and a second limiting position 2611. The first limiting position 2610 is spaced apart from the second limiting position 2611 in the motion direction of the connecting frame 261. When the movable member 171 moves in a first direction, the movable member 171 is capable of pressing the first limiting position 2610 to enable the gland 262 to close and press against the liquid inlet 250. When the movable member 171 moves in a second direction, the movable member 171 is capable of abutting against the second limiting position 2611 to enable the gland 262 to open the liquid inlet 250. The first direction is a direction in which the first reagent needle 16 faces the first reservoir 100 and/or the second reservoir 200, and the second direction is opposite to the first direction.

In this way, through the cooperation of the movable member 171 with the first limiting position 2610 and the second limiting position 2611, the movable member 171 can control the gland 262 to close and press against the liquid inlet 250 or open the liquid inlet 250, such that the liquid inlet 250 can be automatically opened or closed, thereby improving the operational convenience of the fluidic system 1000.

Specifically, the motion direction of the connecting frame 261 is parallel to the direction in which the first reagent needle 16 moves toward the first reservoir 100 and/or the second reservoir 200.

Referring to FIGs. 1, 7, 14, and 15, in some embodiments, the connecting frame 261 is provided with a groove 2613. The groove 2613 extends in the motion direction of the connecting frame 261, and two groove walls of the groove 2613 in the motion direction of the connecting frame 261 form the first limiting position 2610 and the second limiting position 2611, respectively.

In this way, the first limiting position 2610 and the second limiting position 2611 formed by the groove 2613 enable the motion stroke of the connecting frame 261 to be consistent with the motion stroke of the movable member 171, thereby synchronizing the action of extending the first reagent needle 16 into the first reservoir 100 and/or the second reservoir 200 with the action of closing, by the gland 262, the liquid inlet 250.

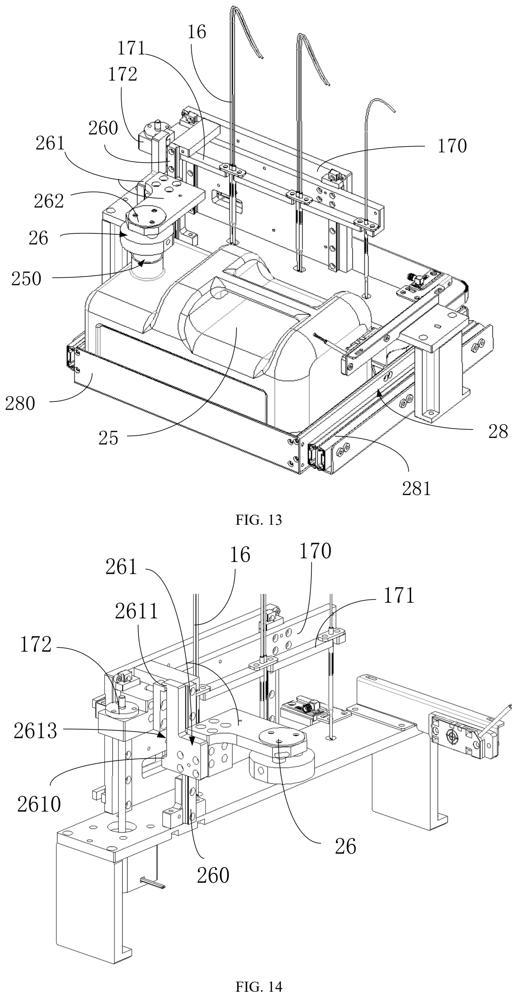

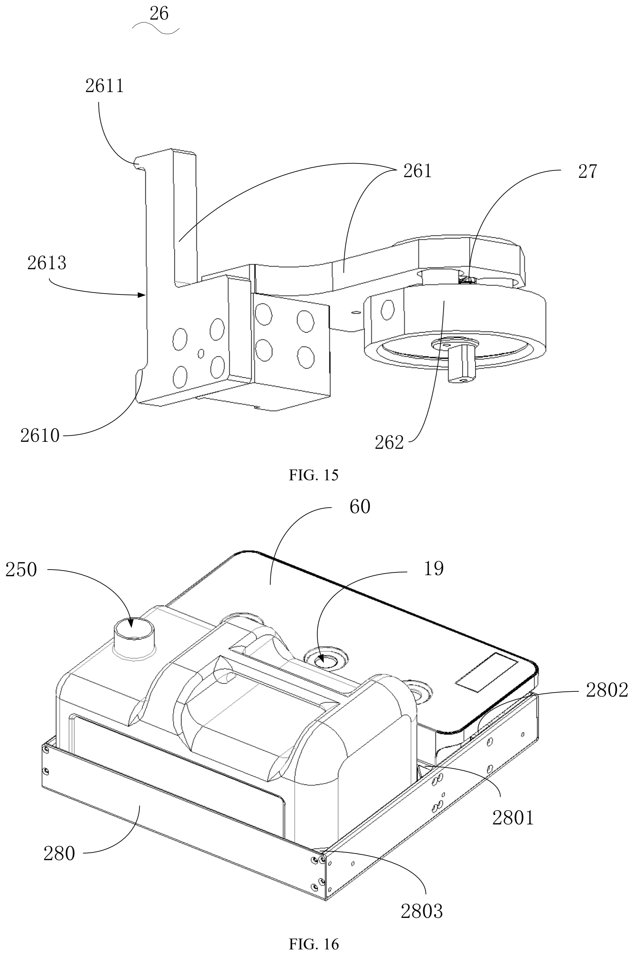

Referring to FIGs. 14 and 15, in some embodiments, an elastic member 27 is arranged between the connecting frame 261 and the gland 262. Two ends of the elastic member 27 are connected to the connecting frame 261 and the gland 262, respectively.

In this way, by connecting the connecting frame 261 to the gland 262 through the elastic member 27, the precision requirement for the gland 262 to close and press against the liquid inlet 250 can be reduced, thereby ensuring that the gland 262 can be in reliable close contact with the liquid inlet 250. It can be understood that if the elastic member 27 is not used, a certain gap may be present when the connecting frame 261 drives the gland 262 to close the liquid inlet 250. In this case, the gland 262 will fail to fully press against the liquid inlet 250, resulting in liquid leakage through the liquid inlet 250.

Specifically, the elastic member 27 may be a standard member such as a compression spring. This is not limited in the embodiments of the present application.

Referring to FIGs. 1, 7, 13, and 16, in some embodiments, the first reservoir 100 and the second reservoir 200 are both located in the same reagent kit 60, and the liquid collector 25 and the reagent kit 60 are both arranged on the same pulling device 28. The pulling device 28 is configured to be arranged in the housing of the fluidic system 1000 by means of pulling.

In this way, by means of pulling, the liquid collector 25 and the reagent kit 60 can be conveniently placed and removed. This arrangement enables the structure within the housing of the fluidic system 1000 to be compact, thereby facilitating management and maintenance.

Specifically, the pulling devices 28 may be spaced apart in the housing of the fluidic system 1000. The pulling device 28 may include a drawer box 280 and sliding rails 281. The drawer box 280 is slidably arranged on the sliding rails 281. The sliding rails 281 are arranged on both sides of the drawer box 280. The drawer box 280 may include a partition plate 2801, a reagent kit poka-yoke block 2802, and a liquid collector poka-yoke block 2803. The partition plate 2801 is configured to separate the liquid collector 25 from the reagent kit 60, the reagent kit poka-yoke block 2802 is configured to prevent incorrect placement of the reagent kit 60, and the liquid collector poka-yoke block 2803 is configured to prevent incorrect placement of the liquid collector 25.

Referring to FIG. 16, in some embodiments, the reagent kit 60 is in a normal temperature state. In this way, the reagents in the reagent kit 60 are stored according to the storage conditions to avoid sample damage.

Referring to FIGs. 1, 7, 9, and 17, in some embodiments, the fluidic system 1000 further includes a bracket 29 and a second driving mechanism 30 connected to the bracket 29. The first reservoir 100 and the second reservoir 200 are both mounted on the bracket 29, and the second driving mechanism 30 is configured to drive the bracket 29 to move, so as to drive the first reservoir 100 and the second reservoir 200 to move. In this way, the movement of the first reservoir 100 and the second reservoir 200 changes the position of the first reagent needle 16 relative to the first reservoir 100 and the second reservoir 200, such that the first reagent needle 16 can aspirate reagents stored at different positions in the first reservoir 100 and the second reservoir 200.

Specifically, the second driving mechanism 30 may be fixedly connected to the bracket 29. The first reservoir 100 and the second reservoir 200 may be detachably mounted on the bracket 29. The second driving mechanism 30 can drive the bracket 29 to move within a plane perpendicular to the motion direction of the first reagent needle 16, thereby enabling the first reservoir 100 and the second reservoir 200 to move within the plane perpendicular to the motion direction of the first reagent needle 16.

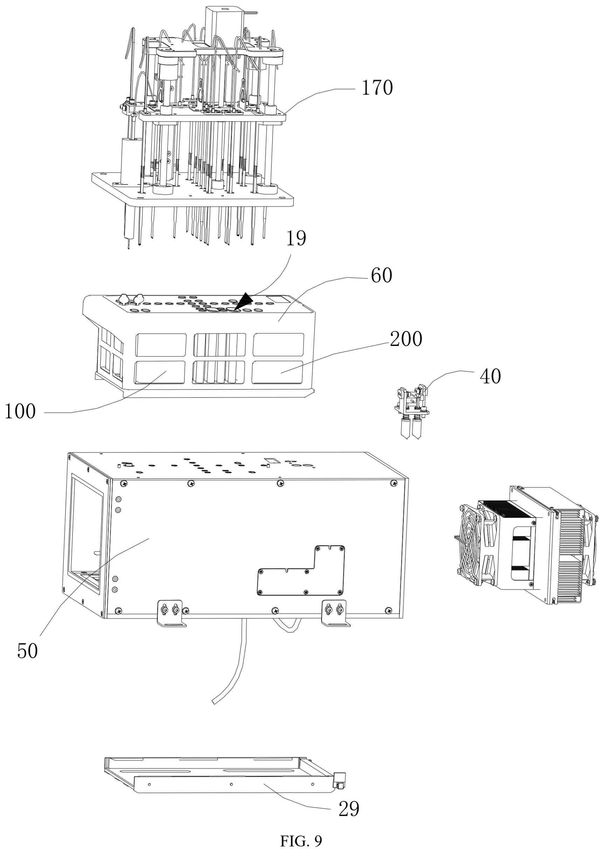

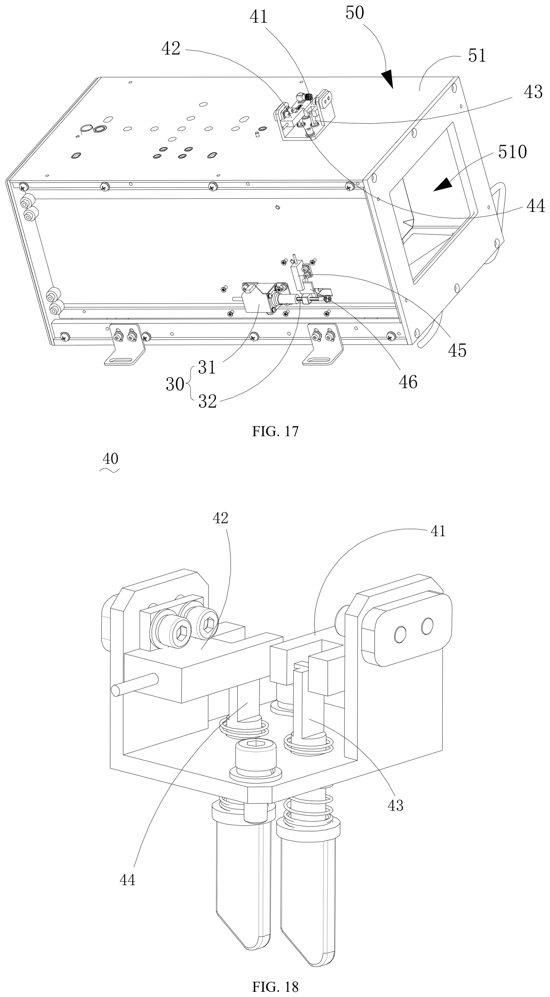

Referring to FIGs. 9 and 17, in some embodiments, the second driving mechanism 30 includes a second motor 31 and a second lead screw 32 connected to the second motor 31. The second lead screw 32 is connected to the bracket 29, and the second motor 31 drives the bracket 29 to move via the second lead screw 32.

In this way, the second motor 31 drives the bracket 29 to move via the second lead screw 32, such that the bracket 29 achieves good position precision and motion repeatability.

Specifically, there may be a plurality of second motors 31 and second lead screws 32, and the plurality of second motors 31 are in a one-to-one correspondence with the second lead screws 32. The plurality of second motors 31 may be spaced apart on both sides of the bracket 29. The bracket 29 may include a connecting rod. The connecting rod may be arranged at the end part of the bracket 29. The second lead screw 32 may be fixedly connected to the connecting rod, such that the movement of the second lead screw 32 drives the bracket 29 to move.

Referring to FIGs. 1, 7, 9, and 17, in some embodiments, the fluidic system 1000 includes a second detection assembly 40. The second detection assembly 40 is configured to detect the position of the bracket 29 to determine the positions of the first reservoir 100 and the second reservoir 200.

In this way, confirming the positions of the first reservoir 100 and the second reservoir 200 can ensure that the first reagent needle 16 can extend into the designated positions within the first reservoir 100 and the second reservoir 200, thereby ensuring that the first reagent needle 16 can accurately aspirate the designated reagent.

Specifically, in the height direction of the first reservoir 100 and the second reservoir 200, the second detection assembly 40 may be arranged on one side of the first reservoir 100 and the second reservoir 200 near the first reagent needle 16.

Referring to FIGs. 9, 17, and 18, in some embodiments, the first reservoir 100 and the second reservoir 200 are both located in the same reagent kit 60. The second detection assembly 40 includes a third photoelectric switch 41, a fourth photoelectric switch 42, a first light blocking member 43, and a second light blocking member 44. The third photoelectric switch 41 and the fourth photoelectric switch 42 are spaced apart above the bracket 29 in the motion direction of the bracket 29. The first light blocking member 43 and the second light blocking member 44 are both capable of coming into contact with the reagent kit 60, and the first light blocking member 43 and the second light blocking member 44 are both capable of moving in a direction perpendicular to the movement direction of the reagent kit 60 when the reagent kit 60 moves. When the reagent kit 60 is in a first position, the first light blocking member 43 blocks the optical path of the third photoelectric switch 41; when the reagent kit 60 is in a second position, the second light blocking member 44 blocks the optical path of the fourth photoelectric switch 42.

In this way, the position of the reagent kit 60 can be determined by determining whether the optical paths of the third photoelectric switch 41 and the fourth photoelectric switch 42 are blocked.

Specifically, the portions of the first light blocking member 43 and the second light blocking member 44 that come into contact with the reagent kit 60 may be designed as inclined surfaces. The surface of the reagent kit 60 that comes into contact with the first light blocking member 43 and the second light blocking member 44 is a first surface. The inclined surfaces form a certain included angle with the first surface of the reagent kit 60. The inclined surfaces, when coming into contact with the reagent kit 60, move in the direction perpendicular to the movement of the reagent kit 60. For example, the liquid aspirated by the first reagent needle 16 is sequencing reagent and cleaning solution. When the bracket 29 drives the reagent kit 60 to move to the first position, the first light blocking member 43 moves in the direction perpendicular to the movement of the reagent kit 60 until the optical path of the third photoelectric switch 41 is blocked by the first light blocking member 43. In this case, it is determined that the first reagent needle 16 is aligned with the second reservoir 200. When the bracket 29 continues to drive the reagent kit 60 to move to the second position, the second light blocking member 44 moves in the direction perpendicular to the movement of the reagent kit 60 until the optical path of the fourth photoelectric switch 42 is blocked by the second light blocking member 44. In this case, it is determined that the first reagent needle 16 is aligned with the first reservoir 100.

Referring to FIGs. 9 and 17, in some embodiments, the first reservoir 100 and the second reservoir 200 are both located in the same reagent kit 60. The second detection assembly 40 includes a fifth photoelectric switch 45 and a third light blocking member 46. The third light blocking member 46 is arranged on the bracket 29. In the case where the bracket 29 is in the initial position, the third light blocking member 46 blocks the optical path of the fifth photoelectric switch 45.

In this way, the cooperation between the fifth photoelectric switch 45 and the third light blocking member 46 can be used to determine whether the reagent kit 60 is placed at the designated position.

Specifically, when the third light blocking member 46 blocks the optical path of the fifth photoelectric switch 45, the reagent kit 60 is placed at the designated position. The designated position refers to the initial position. By changing the position of the bracket 29, the reagent kit 60 can be moved from the initial position to the first position or the second position. In some embodiments, the designated position is the first position of the reagent kit 60. By changing the position of the bracket 29, the reagent kit 60 can be moved from the first position to the second position.

Specifically, the portion of the third light blocking member 46 that comes into contact with the reagent kit 60 may be designed as an inclined surface. The surface of the reagent kit 60 that comes into contact with the third light blocking member 46 is a second surface. The inclined surface forms a certain included angle with the second surface of the reagent kit 60. The inclined surface moves, when coming into contact with the reagent kit 60, moves in the direction perpendicular to the movement of the reagent kit 60.

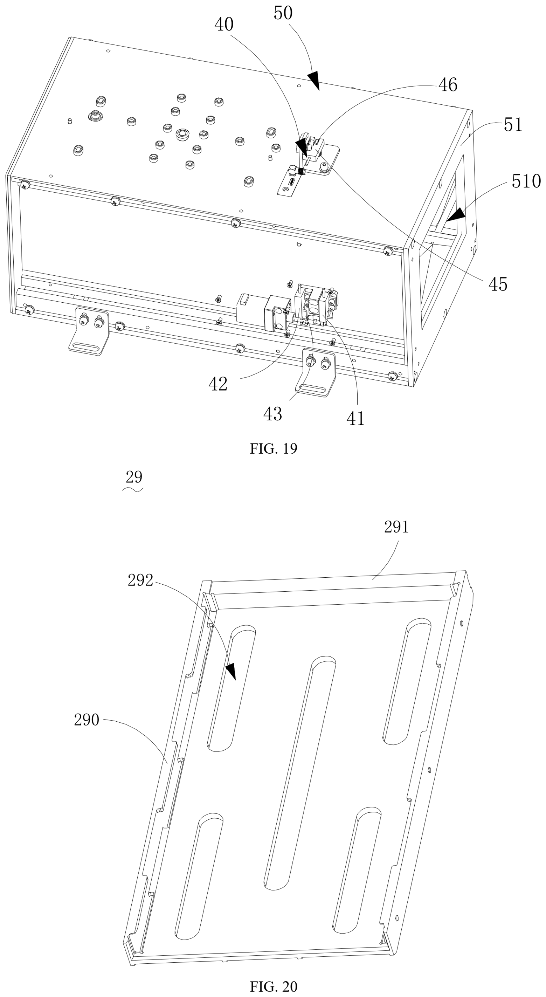

Referring to FIGs. 9 and 19, in some embodiments, the first reservoir 100 and the second reservoir 200 are both located in the same reagent kit 60. The second detection assembly 40 includes a third photoelectric switch 41, a fourth photoelectric switch 42, and a first light blocking member 43. The third photoelectric switch 41 and the fourth photoelectric switch 42 are spaced apart on the side surface of the bracket 29 in the motion direction of the bracket 29, and the first light blocking member 43 is arranged on the bracket 29. The first light blocking member 43 blocks the optical path of the third photoelectric switch 41 when the reagent kit 60 is in a first position, and the first light blocking member 43 blocks the optical path of the fourth photoelectric switch 42 when the reagent kit 60 is in a second position.

In this way, the position of the reagent kit 60 can be determined by determining whether the optical paths of the third photoelectric switch 41 and the fourth photoelectric switch 42 are blocked.

Specifically, the first light blocking member 43 blocks the optical path of the third photoelectric switch 41 when the reagent kit 60 is in the first position. In this case, the reagent kit 60 is in the first position. The first light blocking member 43 blocks the optical path of the fourth photoelectric switch 42 when the reagent kit 60 is in the second position. In this case, the reagent kit 60 is in the second position.

Referring to FIGs. 9 and 19, in some embodiments, the first reservoir 100 and the second reservoir 200 are both located in the same reagent kit 60. The second detection assembly 40 further includes a fifth photoelectric switch 45 and a third light blocking member 46. The third light blocking member 46 is capable of moving in the direction perpendicular to the movement of the reagent kit 60 during the process of placing the reagent kit 60 onto the bracket 29, so as to block the optical path of the fifth photoelectric switch 45. In this way, the cooperation between the fifth photoelectric switch 45 and the third light blocking member 46 can be used to determine whether the reagent kit 60 is placed at the designated position.

Specifically, when the third light blocking member 46 blocks the optical path of the fifth photoelectric switch 45, the reagent kit 60 is placed at the designated position. The designated position refers to the initial position. By changing the position of the bracket 29, the reagent kit 60 can be moved from the initial position to the first position or the second position. In some embodiments, the designated position is the first position of the reagent kit 60. By changing the position of the bracket 29, the reagent kit 60 can be moved from the first position to the second position.

Referring to FIGs. 9 and 20, in some embodiments, the bracket 29 is provided with a limiting structure for limiting the movement of the first reservoir 100 and the second reservoir 200 relative to the bracket 29. In this way, the first reservoir 100 and the second reservoir 200 can be properly placed, such that the first reagent needle 16 can accurately extend into the first reservoir 100 and the second reservoir 200. Specifically, the bracket 29 may include first limiting blocks 290. The first limiting blocks 290 may be arranged on both sides of the bracket 29 in the width direction of the bracket 29. The first limiting blocks 290 are configured to limit the movement of the first reservoir 100 and the second reservoir 200 in the height direction of the bracket 29, thereby prolonging the service life of the first reservoir 100 and the second reservoir 200. The bracket 29 may further include a second limiting block 291. The second limiting block 291 is configured to limit the displacement of the first reservoir 100 and the second reservoir 200 in the length direction of the bracket 29. The second limiting block 291 may be arranged at both ends of the bracket 29 in the length direction.

Referring to FIGs. 8 and 9, in some embodiments, the fluidic system 1000 includes a cooling device 50, and the bracket 29 arranges the first reservoir 100 and the second reservoir 200 in the cooling device 50.

In this way, the cooling device 50 can adjust the temperature of the first reservoir 100 and the second reservoir 200 to a temperature suitable for reagent storage, thereby ensuring the availability of the reagents. Specifically, the cooling device 50 may include a box body 51. The box body 51 may include a chamber 510, the bracket 29 may be fixed in the chamber 510, and the first reservoir 100 and the second reservoir 200 may be accommodated in the chamber 510.

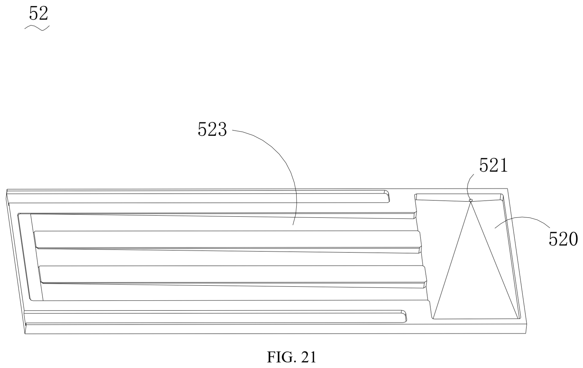

Referring to FIGs. 8, 9, 20, and 21, in some embodiments, the bottom of the bracket 29 is provided with through holes 292 penetrating the bracket 29.

In this way, the through holes 292 can allow the condensed water generated by the cooling device 50 when cooling the first reservoir 100 and the second reservoir 200 to flow to the outside of the bracket 29, thereby avoiding the accumulation of the condensed water and prolonging the service life of the first reservoir 100 and the second reservoir 200. The arrangement of the through holes 292 can also reduce the weight of the bracket 29 and improve the air permeability of the bracket 29.

Specifically, the through holes 292 may be of a regular shape such as a waist-shaped hole or a circular hole, or may be of an irregular shape. The cooling device 50 may further include a lower plate 52. The lower plate 52 may be arranged below the bracket 29 in the height direction of the cooling device 50, and the condensed water in the through holes 292 can flow to the lower plate 52. The lower plate 52 may include an accommodating groove 520. The groove body of the accommodating groove 520 may be configured as a structure that is high on both sides and low in the middle, and the side wall of the accommodating groove 520 is provided with a water discharge hole 521. The lower plate 52 may further include a strip-shaped groove 523. There may be a plurality of strip-shaped grooves 523, and the plurality of strip-shaped grooves 523 are arranged in the length direction of the lower plate 52. The strip-shaped grooves 523 are inclined toward the accommodating groove 520, and the strip-shaped grooves 523 are configured to guide the condensed water flowing from the through holes 292 onto the lower plate 52, such that the condensed water is guided to flow into the accommodating groove 520 and discharged through the water discharge hole 521. The sequencing system according to the embodiments of the present application includes the fluidic system 1000 according to any one of the embodiments above.

In the description of the specification, references to the terms such as "an embodiment", "some embodiments", "schematic embodiments", "examples", "specific examples", or "some examples" mean that the specific feature, structure, material, or characteristic described in connection with the embodiment or example is included in at least one embodiment or example of the present application. In this specification, the schematic description of the above terms does not necessarily refer to the same embodiment or example. Moreover, the particular features, structures, materials, or characteristics described may be combined in any one or more embodiments or examples in any appropriate manner. Although the embodiments of the present application have been illustrated and described, it can be understood by those of ordinary skill in the art that various changes, modifications, replacements, and variations can be made to these embodiments without departing from the principle and purpose of the present application, and the scope of the present application is defined by the claims and equivalents thereof.

## Claims

1. A fluidic system, comprising:
a plurality of reservoirs;
a rotary valve, wherein one of the reservoirs is capable of being in communication with the rotary valve through a first flow path, remaining reservoirs are capable of being in communication with the rotary valve through a second flow path, and the rotary valve is connected to a reaction device through a third flow path; and
a pump assembly, connected to the rotary valve through a fourth flow path, wherein
the fluidic system is selectively in a first state or a second state; when the fluidic system is in the first state, the pump assembly drives liquid in one of the reservoirs to enter the fourth flow path through the rotary valve; and
when the fluidic system is in the second state, the pump assembly drives liquid in at least one of the remaining reservoirs to enter the reaction device through the rotary valve.

2. The fluidic system according to claim 1, wherein the reservoirs comprise a first reservoir and a second reservoir that are independent from each other; the first reservoir is in communication with the rotary valve through the first flow path, and the second reservoir is in communication with the rotary valve through the second flow path.

3. The fluidic system according to claim 2, wherein the first reservoir stores a first solution, and the second reservoir stores a second solution;
when the fluidic system is in the first state, the pump assembly drives the first solution in the first reservoir to enter the fourth flow path through the rotary valve; and
when the fluidic system is in the second state, the pump assembly drives the second solution in the second reservoir to enter the reaction device through the rotary valve.

4. The fluidic system according to claim 3, wherein the first solution is a cleaning solution, and the second solution is a sequencing reagent.

5. The fluidic system according to claim 4, wherein when the fluidic system is in the first state, the pump assembly further drives the cleaning solution in the fourth flow path to flow back to the second flow path to replace a liquid in the second flow path.

6. The fluidic system according to any one of claims 1 to 5, further comprising a multi-way valve arranged between the rotary valve and the reaction device, wherein the multi-way valve changes a path thereof to enable the fluidic system to be in the first state or the second state.

7. The fluidic system according to claim 6, wherein the multi-way valve comprises a first through port, a second through port, and a third through port; the first through port is in selective communication with the second through port or the third through port, and the first through port is in communication with the rotary valve; the second through port is connected to one end of the fourth flow path, and the other end of the fourth flow path is connected to the pump assembly; the third through port is connected to one end of the third flow path, and the other end of the third flow path is connected to the reaction device; and
when the first through port is in communication with the second through port, the fluidic system is in the first state; when the first through port is in communication with the third through port, the fluidic system is in the second state.

8. The fluidic system according to claim 7, wherein the rotary valve comprises a common port and a plurality of connection ports; the common port is in selective communication with at least one of the connection ports, the common port is in communication with the first through port, and the first flow path and the second flow path are connected to corresponding connection ports, respectively;
when the rotary valve is at a first valve position, the common port is in communication with the first flow path via at least one of the connection ports; and
when the rotary valve is at a second valve position, the common port is in communication with the second flow path via remaining connection ports.

9. The fluidic system according to any one of claims 1 to 5, wherein the rotary valve is provided with a first common port, a second common port, a plurality of connection ports, a first communication groove, and a second communication groove; the first common port is connected to one end of the fourth flow path, and the other end of the fourth flow path is connected to the pump assembly; the second common port is connected to one end of the third flow path, and the other end of the third flow path is connected to the reaction device;
in a case where the rotary valve is at a first valve position, the first common port is in communication with the first flow path via at least one of the connection ports and the first communication groove; and
in a case where the rotary valve is at a second valve position, the second common port is in communication with the second flow path via remaining connection ports and the second communication groove.

10. The fluidic system according to claim 9, wherein the rotary valve comprises a stator and a rotor arranged opposite to the stator; the stator is provided with the first common port, the second common port, the plurality of connection ports, and at least a portion of the first communication groove, and the rotor is provided with at least a portion of the second communication groove.

11. The fluidic system according to claim 10, wherein the first communication groove comprises a first groove and a second groove that can communicate with each other, and the first groove and the second groove are provided on the stator and the rotor, respectively.

12. The fluidic system according to claim 11, wherein the first groove is provided with two ends, with one end of the first groove being in communication with the first common port, and the other end being in communication with the second groove; the second groove is provided with two ends, with one end of the second groove being in communication with the first groove and the other end being in selective communication with one of the connection ports.

13. The fluidic system according to claim 12, wherein the stator comprises a first end surface and a second end surface opposite to each other, and the rotor comprises a third end surface and a fourth end surface opposite to each other, wherein the second end surface and the third end surface are in close contact, the first groove is formed on the first end surface, and the second groove is formed on the third end surface.

14. The fluidic system according to claim 10, wherein the second communication groove is provided with two ends, with one end of the second communication groove being in communication with the second common port and the other end being in selective communication with one of the connection ports.

15. The fluidic system according to claim 14, wherein the stator comprises a first end surface and a second end surface opposite to each other, and the rotor comprises a third end surface and a fourth end surface opposite to each other, wherein the second end surface and the third end surface are in close contact, and the second communication groove is formed on the third end surface.

16. The fluidic system according to any one of claims 2 to 15, further comprising a first reagent needle and a first driving mechanism, wherein the first reagent needle is connected to the first flow path and/or the second flow path, and the first driving mechanism is configured to drive the first reagent needle to move in a length direction of the first reagent needle, such that the first reagent needle extends into the first reservoir and/or the second reservoir.

17. The fluidic system according to claim 16, wherein the first driving mechanism comprises:
a fixed frame;
a movable member movably arranged on the fixed frame, wherein the first reagent needle is mounted on the movable member; and
a first driving assembly arranged on the fixed frame, wherein the first driving assembly is configured to drive the movable member to move relative to the fixed frame.

18. The fluidic system according to claim 17, wherein the fixed frame comprises a first plate, a second plate, and connecting posts; the first plate is spaced apart from the second plate, the connecting posts connect the first plate and the second plate, the movable member is movably arranged on the connecting posts in a sleeving manner, and the first driving assembly is mounted on the first plate and the second plate.

19. The fluidic system according to claim 17, wherein the first driving assembly comprises a first motor and a first lead screw connected to the first motor, the movable member is arranged on the first lead screw in a sleeving manner, and the first motor drives the movable member to move via the first lead screw.

20. The fluidic system according to claim 19, wherein the movable member comprises a movable plate arranged on the connecting posts in a sleeving manner and a nut seat arranged on the first lead screw in a sleeving manner; the nut seat is in a threaded connection with the first lead screw, and the nut seat is fixedly connected to the movable plate.

21. The fluidic system according to claim 17, further comprising a first detection assembly arranged on the fixed frame, wherein the first detection assembly is configured to detect a position of the movable member.

22. The fluidic system according to claim 21, wherein the first detection assembly comprises a first photoelectric switch and a second photoelectric switch spaced apart in a motion direction of the movable member; the first photoelectric switch and the second photoelectric switch are configured to detect an upper limit position and a lower limit position of the movable member, respectively.

23. The fluidic system according to any one of claims 16 to 22, further comprising a second reagent needle and a mounting base, wherein the mounting base is mounted on the fixed frame, the second reagent needle is connected to the first flow path and/or the second flow path, the second reagent needle is mounted on the mounting base, and the movable member, when moving toward the first reservoir and/or the second reservoir, drives the second reagent needle to extend into the first reservoir and/or the second reservoir.

24. The fluidic system according to claim 23, wherein the mounting base comprises a guiding member and a sliding member; the guiding member is mounted on the fixed frame, the sliding member is movably arranged on the guiding member, the second reagent needle is mounted on the sliding member, and the movable member, when moving toward the first reservoir and/or the second reservoir, drives the sliding member to move.

25. The fluidic system according to claim 24, wherein a first elastic member is arranged between the sliding member and the fixed frame; the first elastic member is compressed to store energy when the movable member moves toward the first reservoir and/or the second reservoir, and releases the stored energy after a force between the movable member and the sliding member is eliminated, so as to move the second reagent needle outward from the first reservoir and/or the second reservoir.

26. The fluidic system according to claim 24, wherein a second elastic member is connected between the sliding member and the second reagent needle; the second elastic member applies a force in a first direction to the second reagent needle so as to keep the second reagent needle on the sliding member; when the second reagent needle is subjected to a force in a second direction, the second elastic member is compressed under an action of the second reagent needle, wherein the first direction is a direction in which the second reagent needle faces the first reservoir and/or the second reservoir, and the second direction is opposite to the first direction.

27. The fluidic system according to claim 26, wherein the sliding member is provided with a mounting hole, and the second reagent needle comprises a needle body and a flange arranged on the needle body, wherein the needle body passes through the sliding member, the flange is arranged in the mounting hole, and the second elastic member abuts against the flange.

28. The fluidic system according to any one of claims 16 to 22, further comprising a liquid collector configured to collect a liquid generated by the fluidic system, wherein the liquid collector is provided with a liquid inlet, and the fluidic system further comprises a cover assembly;
the first driving mechanism drives the cover assembly to close and press against the liquid inlet in a process of driving the first reagent needle to extend into the first reservoir and/or the second reservoir; or
the first driving mechanism drives the cover assembly to open the liquid inlet in a process of driving the first reagent needle to exit the first reservoir and/or the second reservoir.

29. The fluidic system according to claim 28, wherein the first driving mechanism comprises:
a fixed frame;
a movable member movably arranged on the fixed frame, wherein the first reagent needle is mounted on the movable member; and
a first driving assembly arranged on the fixed frame, wherein the first driving assembly is configured to drive the movable member to move relative to the fixed frame, and during the movement of the movable member, the movable member is capable of driving the cover assembly to enable the cover assembly to close and press against the liquid inlet or open the liquid inlet.

30. The fluidic system according to claim 29, wherein the cover assembly comprises a guide rail, a connecting frame, and a gland; the guide rail is mounted on the fixed frame, the connecting frame is movably arranged on the guide rail, and the gland is arranged on the connecting frame; during the movement of the movable member, the movable member is capable of driving the connecting frame to enable the gland to close and press against the liquid inlet or open the liquid inlet.

31. The fluidic system according to claim 30, wherein the connecting frame is provided with a first limiting position and a second limiting position; the first limiting position is spaced apart from the second limiting position in a motion direction of the connecting frame; when the movable member moves in a first direction, the movable member is capable of pressing the first limiting position to enable the gland to close and press against the liquid inlet; when the movable member moves in a second direction, the movable member is capable of abutting against the second limiting position to enable the gland to open the liquid inlet, wherein the first direction is a direction in which the first reagent needle faces the first reservoir and/or the second reservoir, and the second direction is opposite to the first direction.

32. The fluidic system according to claim 31, wherein the connecting frame is provided with a groove; the groove extends in the motion direction of the connecting frame, and two groove walls of the groove in the motion direction of the connecting frame form the first limiting position and the second limiting position, respectively.

33. The fluidic system according to claim 30, wherein an elastic member is arranged between the connecting frame and the gland; two ends of the elastic member are connected to the connecting frame and the gland, respectively.

34. The fluidic system according to claim 28, wherein the first reservoir and the second reservoir are both located in a same reagent kit, and the liquid collector and the reagent kit are both arranged on a same pulling device, wherein the pulling device is configured to be arranged in a housing of the fluidic system by means of pulling.

35. The fluidic system according to claim 34, wherein the reagent kit is in a normal temperature state.

36. The fluidic system according to any one of claims 2 to 35, further comprising a bracket and a second driving mechanism connected to the bracket, wherein the first reservoir and the second reservoir are both mounted on the bracket, and the second driving mechanism is configured to drive the bracket to move, so as to drive the first reservoir and the second reservoir to move.

37. The fluidic system according to claim 36, wherein the second driving mechanism comprises a second motor and a second lead screw connected to the second motor; the second lead screw is connected to the bracket, and the second motor drives the bracket to move via the second lead screw.

38. The fluidic system according to claim 36, further comprising a second detection assembly, wherein the second detection assembly is configured to detect a position of the bracket to determine positions of the first reservoir and the second reservoir.

39. The fluidic system according to claim 38, wherein the first reservoir and the second reservoir are both located in the same reagent kit, and the second detection assembly comprises a third photoelectric switch, a fourth photoelectric switch, a first light blocking member, and a second light blocking member, wherein the third photoelectric switch and the fourth photoelectric switch are spaced apart above the bracket in a motion direction of the bracket, the first light blocking member and the second light blocking member are both capable of coming into contact with the reagent kit, and the first light blocking member and the second light blocking member are both capable of moving in a direction perpendicular to the movement of the reagent kit when the reagent kit moves; when the reagent kit is in a first position, the first light blocking member blocks an optical path of the third photoelectric switch; when the bracket is in a second position, the second light blocking member blocks an optical path of the fourth photoelectric switch.

40. The fluidic system according to claim 39, wherein portions of the first light blocking member and the second light blocking member that come into contact with the reagent kit are inclined surfaces, a surface of the reagent kit that comes into contact with the first light blocking member and the second light blocking member is a first surface, and the inclined surfaces form a certain included angle with the first surface.

41. The fluidic system according to claim 39, wherein the first reservoir and the second reservoir are both located in the same reagent kit, and the second detection assembly further comprises a fifth photoelectric switch and a third light blocking member, wherein the third light blocking member is arranged on the bracket, and in a case where the reagent kit is in an initial position, the third light blocking member blocks an optical path of the fifth photoelectric switch.

42. The fluidic system according to claim 38, wherein the first reservoir and the second reservoir are both located in the same reagent kit, and the second detection assembly comprises a third photoelectric switch, a fourth photoelectric switch, and a first light blocking member, wherein the third photoelectric switch and the fourth photoelectric switch are spaced apart on a side surface of the bracket in a motion direction of the bracket, the first light blocking member is arranged on the bracket, the first light blocking member blocks an optical path of the third photoelectric switch when the reagent kit is in a first position, and the first light blocking member blocks an optical path of the fourth photoelectric switch when the reagent kit is in a second position.

43. The fluidic system according to claim 42, wherein the first reservoir and the second reservoir are both located in the same reagent kit, and the second detection assembly further comprises a fifth photoelectric switch and a third light blocking member, wherein the third light blocking member is capable of moving in a direction perpendicular to the movement of the reagent kit during a process of placing the reagent kit onto the bracket, so as to block an optical path of the fifth photoelectric switch.

44. The fluidic system according to claim 43, wherein a portion of the third light blocking member that comes into contact with the reagent kit is an inclined surface, a surface of the reagent kit that comes into contact with the third light blocking member is a second surface, and the inclined surface forms a certain included angle with the second surface.

45. The fluidic system according to any one of claims 36 to 44, wherein the bracket is provided with a limiting structure for limiting the movement of the first reservoir and the second reservoir relative to the bracket.

46. The fluidic system according to any one of claims 36 to 45, comprising a cooling device, wherein the bracket arranges the first reservoir and the second reservoir in the cooling device.

47. The fluidic system according to claim 46, wherein a bottom of the bracket is provided with through holes penetrating the bracket.

48. A sequencing system, comprising the fluidic system according to any one of claims 1 to 47.
